Europäisches Patentamt

European Patent Office

Office européen des brevets

(19)

(11) **EP 1 491 207 A1**

(12) **EUROPEAN PATENT APPLICATION**
published in accordance with Art. 158(3) EPC

(43) Date of publication:
**29.12.2004 Bulletin 2004/53**

(21) Application number: **03715507.4**

(22) Date of filing: **27.03.2003**

(51) Int Cl.[7]: **A61K 38/17**, A61K 39/395,
A61K 45/00, A61K 48/00,
A61P 9/00, A61P 11/00,
A61P 13/00, A61P 15/00,
A61P 21/00, A61P 25/00,
A61P 43/00

(86) International application number:
**PCT/JP2003/003828**

(87) International publication number:
**WO 2003/082320 (09.10.2003 Gazette 2003/41)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **28.03.2002 JP 2002093045**
**13.12.2002 JP 2002361580**

(71) Applicant: **Takeda Chemical Industries, Ltd.**
**Osaka-shi, Osaka 541-0045 (JP)**

(72) Inventors:
 • **ITO, Yasuaki**
  **Tsuchiura-shi, Ibaraki 300-0832 (JP)**

 • **SHINOHARA, Tokuyuki**
  **Tsukuba-shi, Ibaraki 305-0047 (JP)**
 • **HOSOYA, Masaki**
  **Tsuchiura-shi, Ibaraki 300-0007 (JP)**
 • **HINUMA, Shuji**
  **Tsukuba-shi, Ibaraki 305-0821 (JP)**
 • **NOGUCHI, Yuko**
  **Tsukuba-shi, Ibaraki 305-0051 (JP)**

(74) Representative: **Rickard, Timothy Mark Adrian**
**Takeda Euro IP Department,**
**11-12 Charles II Street**
**London SW1Y 4QU (GB)**

(54) **NOVEL SCREENING METHOD**

(57)     Using a novel G protein-coupled receptor protein having an amino acid sequence which is the same or substantially the same amino acid sequence as an amino acid sequence represented by SEQ ID NO: 2 or its salt together with β-alanine or L-carnosine, an agonist or an antagonist to the receptor protein or its salt can be efficiently screened.

EP 1 491 207 A1

**Description**

TECHNICAL FIELD

The present invention relates to use of human- and rat-derived G protein-coupled receptor proteins (TGR7 and rCB7T084).

BACKGROUND ART

Physiological active substances such as various hormones, neurotransmitters, etc. regulate the biological function via specific receptor proteins present on cell membranes. Many of these receptor proteins are coupled with guanine nucleotide-binding protein (hereinafter sometimes simply referred to as G protein) and mediate the intracellular signal transduction via activation of G protein. These receptor proteins possess the common structure containing seven transmembrane domains and are thus collectively referred to as G protein-coupled receptor proteins (GPCR) or seven-transmembrane receptor proteins (7TMR).

G protein-coupled receptor proteins are present on the cell surface of each functional cell and organ in the body, and play important physiological roles as the target of the molecules that regulate the functions of the cells and organs, e.g., hormones, neurotransmitters, physiologically active substances and the like. Receptor proteins transmit signals to cells via binding with physiologically active substances, and the signals induce various reactions such as activation and inhibition of the cells.

To clarify the relationship between substances that regulate complex biological functions in various cells and organs in the body, and their specific receptor proteins, in particular, G protein-coupled receptor proteins would elucidate the functional mechanisms in various cells and organs in the body to provide a very important means for development of drugs closely associated with these functions.

For example, in various organs, their physiological functions are controlled in vivo through regulation by many hormones, hormone-like substances, neurotransmitters or physiologically active substances. In particular, physiologically active substances are found in numerous sites of the body and regulate the physiological functions through their corresponding receptor proteins. Many unknown hormones, neurotransmitters or many other physiologically active substances still exist in the body and, as to their receptor proteins, many of these proteins have not yet been reported. In addition, it is still unknown if there are subtypes of known receptor proteins.

It is very important for development of drugs to clarify the relationship between substances that regulate elaborated functions in vivo and their specific receptor proteins. Furthermore, for efficient screening of agonists and antagonists to receptor proteins in development of drugs, it is required to clarify functional mechanisms of receptor protein genes expressed in vivo and express the genes in an appropriate expression system.

In recent years, random analysis of cDNA sequences has been actively studied as a means for analyzing genes expressed in vivo. The sequences of cDNA fragments thus obtained have been registered on and published to databases as Expressed Sequence Tag (EST). However, since many ESTs contain sequence information only, it is difficult to predict their functions.

The amino acid sequence of rat-derived rCB7T084 and the DNA encoding the same are reported (Japanese Patent Laid-Open Application No. 2000-17569). Furthermore, the amino acid sequence of human-derived TGR7 having a high homology to rCB7T084 and the DNA encoding the same are reported (WO 01/83748, WO 01/66750, WO 01/48188, WO 01/36471, WO 01/57085 and WO 01/70814). However, functions of these G protein-coupled receptor proteins and their physiological ligands remain unresolved.

Heretofore, substances that inhibit the binding of G protein-coupled receptor proteins to physiologically active substances (i.e., ligands) and substances that bind and induce signals similar to those physiologically active substances (i.e., ligands) have been utilized for pharmaceuticals as antagonists and agonists specific to these receptor proteins that regulate the biological functions. Therefore, it is a very important means in search for agonists and antagonists that can be targeted for pharmaceutical development to determine specific ligands to G protein-coupled receptor proteins.

However, many G protein-coupled receptor proteins with unknown functions and many so-called orphan receptor proteins in which the corresponding ligands are yet unidentified are present even at this point of time. Thus, search of ligands to G protein-coupled receptor proteins and elucidation of their functions are eagerly awaited.

G protein-coupled receptor proteins are useful in search for a novel physiological active substance (i.e., ligand) using the signal transduction activity as an indicator and in search for agonists and antagonists of the receptor protein. On the other hand, even though no physiological ligand is found, agonists and antagonist of the receptor protein may be prepared by analyzing the physiological action of the receptor protein through inactivation experiment of the receptor protein (knockout animal). Ligands, agonists or antagonists, etc. of these receptor proteins are expected to be utilized as preventive/therapeutic agents and diagnostic agents for diseases associated with dysfunction or hyperfunction of the G protein-coupled receptor proteins.

Attenuation or accentuation in functions of G protein-coupled receptor proteins due to genetic aberration of the G protein-coupled receptor proteins in vivo causes some disorders in many cases. In this case, the G protein coupled receptor proteins may be used not only for administration of antagonists or agonists to the receptor proteins, but also for gene therapy by transfer of the receptor protein gene into the body (or some particular organs) or by introduction of the antisense nucleic acid of the receptor protein gene. In this case, information on the base sequence of the receptor protein is essentially required for investigating deletion or mutation on the gene. The receptor protein gene is also applicable as preventive/therapeutic agents and diagnostic agents for diseases associated with dysfunction of the receptor protein.

The present invention relates to determination of ligands to orphan G protein-coupled receptor proteins with unknown functions and use of the G protein-coupled receptor proteins and ligands thereto. That is, the present invention aims at providing a method of screening a compound (antagonist, agonist) or its salt that changes the binding properties of the ligand to the G protein-coupled receptor protein; a kit for the screening; a compound (antagonist, agonist) or its salt that changes the binding properties of the ligand to the G protein-coupled receptor protein a compound (antagonist, agonist) or its salt that changes the binding properties of the ligand to the G protein-coupled receptor protein, which is obtainable by using the screening method or the screening kit; a pharmaceutical comprising a compound (antagonist, agonist) that changes the binding properties of the ligand to the G protein-coupled receptor protein or a compound (antagonist, agonist) that changes the expression level of the G protein-coupled receptor protein, or a salt thereof; and the like.

DISCLOSURE OF THE INVENTION

The present inventors made extensive studies and as a result, found that ligands to rat-derived rCB7T084 and human-derived TGR7 are β-alanine or L-carnosine. Based on these findings, the present inventors continued further studies and have come to accomplish the present invention.

That is, the present invention provides the following features, and the like.

[1] A central or peripheral neural function-regulating agent comprising a G protein-coupled receptor protein containing the same or substantially the same amino acid sequence as the amino acid sequence represented by SEQ ID NO: 2 or SEQ ID NO: 4, its partial peptide, or a salt thereof;

[2] An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising a G protein-coupled receptor protein containing the same or substantially the same amino acid sequence as the amino acid sequence represented by SEQ ID NO: 2 or SEQ ID NO: 4, its partial peptide, or a salt thereof;

[3] A central or peripheral neural function-regulating agent comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide;

[4] An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide;

[5] A diagnostic agent for central or peripheral neural function abnormalities comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide;

[6] A diagnostic agent for anxiety, insomnia, excitement, muscular atrophy, anesthesia sensitivity, convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia, palsy, ataxia, paralysis, depression, delayed awakening, deficits in memory and learning, hypotension, dysuria, hypertrophy of bladder, dyspnea or sensory paralysis, comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide;

[7] A central or peripheral neural function-regulating agent comprising an antibody to a G protein-coupled receptor protein having the same or substantially the same amino acid sequence as the amino acid sequence represented by SEQ ID NO: 2 or SEQ ID NO: 4, its partial peptide, or a salt thereof;

[8] A preventive/therapeutic agent for ataxia, paralysis, depression, delayed awakening, deficits in memory and learning, hypotension, dysuria, hypertrophy of bladder, dyspnea, dependence or sensory paralysis, comprising an antibody to a G protein-coupled receptor protein having the same or substantially the same amino acid sequence as the amino acid sequence represented by SEQ ID NO: 2 or SEQ ID NO: 4, its partial peptide, or a salt thereof;

[9] A central or peripheral neural function-regulating agent comprising a polynucleotide comprising a base sequence complementary to a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence;

[10] A preventive/therapeutic agent for ataxia, paralysis, depression, delayed awakening, deficits in memory and learning, hypotension, dysuria, hypertrophy of bladder, dyspnea, dependence or sensory paralysis, comprising a polynucleotide comprising a base sequence complementary to a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence;

[11] A method of screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine or L-carnosine, which comprises using (1) the receptor protein, its partial peptide, or a salt thereof and (2) β-alanine or L-carnosine;

[12] A kit for screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine or L-carnosine, comprising (1) the receptor protein, its partial peptide, or a salt thereof and (2) β-alanine or L-carnosine;

[13] A compound or its salt that changes the binding properties of β-alanine or L-carnosine to a G protein-coupled receptor protein containing the same or substantially the same amino acid sequence as the amino acid sequence represented by SEQ ID NO: 2 or SEQ ID NO: 4, or a salt thereof, which is obtainable using the screening method according to [11] or the screening kit according to [12];

[14] A method of screening an agonist or antagonist to a G protein-coupled receptor protein containing the same or substantially the same amino acid sequence as the amino acid sequence represented by SEQ ID NO: 2 or SEQ ID NO: 4, or a salt thereof, which comprises using (1) the receptor protein, its partial peptide, or a salt thereof and (2) a compound or its salt that changes the binding properties of the receptor protein or a salt thereof to β-alanine or L-carnosine;

[15] A kit for screening an agonist or antagonist to a G protein-coupled receptor protein containing the same or substantially the same amino acid sequence as the amino acid sequence represented by SEQ ID NO: 2 or SEQ ID NO: 4, or a salt thereof, comprising (1) the receptor protein, its partial peptide, or a salt thereof and (2) a compound or its salt that changes the binding properties of the receptor protein or a salt thereof to β-alanine or L-carnosine;

[16] A pharmaceutical comprising a compound or its salt that changes the binding properties of β-alanine or L-carnosine to a G protein-coupled receptor protein containing the same or substantially the same amino acid sequence as the amino acid sequence represented by SEQ ID NO: 2 or SEQ ID NO: 4, or a salt thereof;

[17] The pharmaceutical according to [16], which is a central or peripheral neural function-regulating agent;

[18] A method of screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine, which comprises using (1) the receptor protein, its partial peptide, or a salt thereof and (2) β-alanine;

[19] A kit for screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine, comprising (1) the receptor protein, its partial peptide, or a salt thereof and (2) β-alanine;

[20] A compound or its salt that changes the binding properties of β-alanine to a G protein-coupled receptor protein containing the same or substantially the same amino acid sequence as the amino acid sequence represented by SEQ ID NO: 2 or SEQ ID NO: 4, or a salt thereof, which is obtainable using the screening method according to [18] or the screening kit according to [19];

[21] A method of screening an agonist or antagonist to a G protein-coupled receptor protein containing the same or substantially the same amino acid sequence as the amino acid sequence represented by SEQ ID NO: 2 or SEQ ID NO: 4, or a salt thereof, which comprises using (1) the receptor protein, its partial peptide, or a salt thereof and (2) a compound or its salt that changes the binding properties of the receptor protein or a salt thereof to β-alanine;

[22] A kit for screening an agonist or antagonist to a G protein-coupled receptor protein containing the same or substantially the same amino acid sequence as the amino acid sequence represented by SEQ ID NO: 2 or SEQ ID NO: 4, or a salt thereof, comprising (1) the receptor protein, its partial peptide, or a salt thereof and (2) a compound or its salt that changes the binding properties of the receptor protein or a salt thereof to β-alanine;

[23] A pharmaceutical comprising a compound or its salt that changes the binding properties of β-alanine to a G protein-coupled receptor protein containing the same or substantially the same amino acid sequence as the amino acid sequence represented by SEQ ID NO: 2 or SEQ ID NO: 4, or a salt thereof;

[24] The pharmaceutical according to [16], which is a central or peripheral neural function-regulating agent;

[25] An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising an agonist to a G protein-coupled receptor protein containing the same or substantially the same amino acid sequence as the amino acid sequence represented by SEQ ID NO: 2 or SEQ ID NO: 4, or a salt thereof;

[26] A preventive/therapeutic agent for ataxia, paralysis, depression, delayed awakening, deficits in memory and learning, hypotension, dysuria, hypertrophy of bladder, dyspnea, dependence or sensory paralysis, comprising an antagonist to a G protein-coupled receptor protein containing the same or substantially the same amino acid sequence as the amino acid sequence represented by SEQ ID NO: 2 or SEQ ID NO: 4, or a salt thereof;

[27] A method of screening a compound having an action of changing the expression level of a G protein-coupled receptor protein having the same or substantially the same amino acid sequence as the amino acid sequence represented by SEQ ID NO: 2 or SEQ ID NO: 4 to regulate the central or peripheral neural function, which comprises using a polynucleotide containing a polynucleotide encoding the G protein-coupled receptor protein or its partial peptide;

[28] A kit for screening a compound having an action of changing the expression level of a G protein-coupled receptor protein having the same or substantially the same amino acid sequence as the amino acid sequence represented by SEQ ID NO: 2 or SEQ ID NO: 4 to regulate the central or peripheral neural function, comprising a polynucleotide containing a polynucleotide encoding the G protein-coupled receptor protein or its partial peptide;

[29] A compound having an action of changing the expression level of a G protein-coupled receptor protein having the same or substantially the same amino acid sequence as the amino acid sequence represented by SEQ ID NO: 2 or SEQ ID NO: 4 or its partial peptide to regulate the central or peripheral neural function, which is obtainable by using the screening method according to [27] or the screening kit according to [28];

[30] A central or peripheral neural function-regulating agent comprising a compound which changes the expression level of a G protein-coupled receptor protein having the same or substantially the same amino acid sequence as the amino acid sequence represented by SEQ ID NO: 2 or SEQ ID NO: 4 or its partial peptide;

[31] An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising a compound or its salt that increases the expression level of a G protein-coupled receptor protein containing the same or substantially the same amino acid sequence as the amino acid sequence represented by SEQ ID NO: 2 or SEQ ID NO: 4, or its partial peptide;

[32] A preventive/therapeutic agent for ataxia, paralysis, depression, delayed awakening, deficits in memory and learning, hypotension, dysuria, hypertrophy of bladder, dyspnea, dependence or sensory paralysis, comprising a compound or its salt that decreases the expression level of a G protein-coupled receptor protein containing the same or substantially the same amino acid sequence as the amino acid sequence represented by SEQ ID NO: 2 or SEQ ID NO: 4, or its partial peptide;

[33] A method of screening an agonist to a G protein-coupled receptor protein containing the same or substantially the same amino acid sequence as the amino acid sequence represented by SEQ ID NO: 2 or SEQ ID NO: 4, or a salt thereof, which comprises measuring the intracellular cAMP production suppressing activity when a test compound is brought in contact with a cell containing the receptor protein;

[34] An agent for fortifying the signal transduction activity of a G protein-coupled receptor protein containing the same or substantially the same amino acid sequence as the amino acid sequence represented by SEQ ID NO: 2 or SEQ ID NO: 4, comprising β-alanine or L-carnosine;

[35] The agent according to [34], which is a central or peripheral neural function-regulating agent;

[36] The agent according to [34], which is an antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy;

[37] An agent for fortifying the signal transduction activity of a G protein-coupled receptor protein containing the same or substantially the same amino acid sequence as the amino acid sequence represented by SEQ ID NO: 2 or SEQ ID NO: 4, comprising β-alanine;

[38] The agent according to [37], which is a central or peripheral neural function-regulating agent;

[39] The agent according to [37], which is an antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy;

[40] A method for antianxiety, hypnosis and sedation, muscle relaxation or anesthesia enhancement, or for the prevention/treatment of convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, which comprises administering to a mammal an effective dose of (1) a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or (3) an agonist to a G protein-coupled receptor protein having the same or substantially the same amino acid sequence as the amino acid sequence represented by SEQ ID NO: 2 or SEQ ID NO: 4, or a salt thereof;

[41] A method of preventing/treating ataxia, paralysis, depression, delayed awakening, deficits in memory and learning, hypotension, dysuria, hypertrophy of bladder, dyspnea, dependence or sensory paralysis, which comprises administering to a mammal an effective dose of (1) an antibody to a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence, or (3) an antagonist to a G protein-coupled receptor protein having the same or substantially the same amino acid sequence as the amino acid sequence represented by SEQ ID NO: 2 or SEQ ID NO: 4, or a salt thereof;

[42] Use of (1) a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or (3) an agonist to a G protein-coupled receptor protein having the same or substantially the same amino acid sequence as the amino acid sequence represented by SEQ ID NO: 2 or SEQ ID NO: 4, to manufacture an antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy;

[43] Use of (1) an antibody to a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide comprising a base sequence complementary to a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence, or (3) an antagonist to a G protein-coupled receptor protein having the same or substantially the same amino acid sequence as the amino acid sequence represented by SEQ ID NO: 2 or SEQ ID NO: 4, to manufacture a preventive/therapeutic agent for ataxia, paralysis, depression, delayed awakening, deficits in memory and learning, hypotension, dysuria, hypertrophy of bladder, dyspnea, dependence or sensory paralysis;

[44] A diagnostic agent for central or peripheral neural function abnormalities comprising an antibody to a G protein-coupled receptor protein having the same or substantially the same amino acid sequence as the amino acid sequence represented by SEQ ID NO: 2 or SEQ ID NO: 4, its partial peptide, or a salt thereof;

[45] A diagnostic agent for anxiety, insomnia, excitement, muscular atrophy, anesthesia sensitivity, convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia, palsy, ataxia, paralysis, depression, delayed awakening, deficits in memory and learning, hypotension, dysuria, hypertrophy of bladder, dyspnea or sensory paralysis, comprising an antibody to a G protein-coupled receptor protein having the same or substantially the same amino acid sequence as the amino acid sequence represented by SEQ ID NO: 2 or SEQ ID NO: 4, its partial peptide, or a salt thereof.

The present invention further provides the following features.

[46] The screening method according to [11], wherein comparison is made between (i) the case where a G protein-coupled receptor protein containing the same or substantially the same amino acid sequence as the amino acid sequence represented by SEQ ID NO: 2 or SEQ ID NO: 4 (hereinafter sometimes referred to as the GPCR of the invention), its partial peptide, or a salt thereof is brought in contact with β-alanine or L-carnosine and (ii) the case where the GPCR of the invention, its partial peptide, or a salt thereof is brought in contact with β-alanine or L-carnosine and a test compound;

[47] The screening method according to [11], wherein the binding amount of labeled β-alanine or L-carnosine to the GPCR of the invention, its partial peptide, or a salt thereof is determined in (i) the case where said labeled β-alanine or L-carnosine is brought in contact with the GPCR of the invention, its partial peptide, or a salt thereof and (ii) the case where the labeled β-alanine or L-carnosine and a test compound are brought in contact with the GPCR of the invention, its partial peptide, or a salt thereof, followed by comparing the binding amount between the cases;

[48] The screening method according to [11], wherein the binding amount of labeled β-alanine or L-carnosine to a cell containing the GPCR of the invention is determined in (i) the case where the labeled β-alanine or L-carnosine is brought in contact with the cell containing the GPCR of the invention and (ii) the case where the labeled β-alanine or L-carnosine and a test compound are brought in contact with the cell containing the GPCR of the invention, followed by comparing the binding amount between the cases;

[49] The screening method according to [11], wherein the binding amount of labeled β-alanine or L-carnosine to a membrane fraction of cell containing the GPCR of the invention is determined in (i) the case where the labeled β-alanine or L-carnosine is brought in contact with the membrane fraction of cell containing the GPCR of the invention and (ii) the case where the labeled β-alanine or L-carnosine and a test compound are brought in contact with the membrane fraction of cell containing the GPCR of the invention, followed by comparing the binding amount between the cases;

[50] The screening method according to [11], wherein the binding amount of labeled β-alanine or L-carnosine to the GPCR of the invention is determined in (i) the case where the labeled β-alanine or L-carnosine is brought in contact with the GPCR of the invention expressed on a cell membrane of a transformant by culturing the transformant transformed with a recombinant vector having a DNA containing a DNA encoding the GPCR of the invention and (ii) the case where the labeled β-alanine or L-carnosine and a test compound are brought in contact with the GPCR of the invention expressed on a cell membrane of a transformant by culturing the transformant transformed with a recombinant vector having a DNA containing a DNA encoding the GPCR of the invention, followed by comparing the binding amount between the cases;

[51] The screening method according to [11], wherein a cell stimulating activity mediated by the GPCR of the invention is determined in (i) the case where a compound activating the GPCR of the invention is brought in contact with a cell containing the GPCR of the invention and (ii) the case where a compound activating the GPCR of the invention and a test compound are brought in contact with the cell containing the GPCR of the invention, followed by comparing the activity between the cases;

[52] The screening method according to [11], wherein a cell stimulating activity mediated by the GPCR of the invention is determined in (i) the case where a compound activating the GPCR of the invention is brought in contact with the GPCR of the invention expressed on a cell membrane of a transformant by culturing the transformant transformed with a recombinant vector having a DNA containing a DNA encoding the GPCR of the invention and (ii) the case where the compound activating the GPCR of the invention and a test compound are brought in contact with the GPCR of the invention expressed on a cell membrane of a transformant by culturing the transformant transformed with a recombinant vector having a DNA containing a DNA encoding the GPCR of the invention, followed by comparing the activity between the cases;

[53] The screening method according to [51] or [52], wherein the compound activating the GPCR of the invention is β-alanine or L-carnosine;

[54] The screening kit according to [12], which comprises a cell containing the GPCR of the invention or its membrane fraction;

[55] The screening kit according to [12], which comprises the GPCR of the invention expressed on the cell membrane of said transformant by culturing the transformant transformed with a recombinant vector having a DNA containing a DNA encoding the GPCR of the invention;

[56] A method of confirming that a central or peripheral neural function-regulating drug or a preventive/therapeutic drug for anxiety, insomnia, excitement, muscular atrophy, anesthesia sensitivity, convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia, palsy, ataxia, paralysis, depression, delayed awakening, deficits in memory and learning, hypotension, dysuria, hypertrophy of bladder, dyspnea or sensory paralysis binds to a G protein-coupled receptor protein containing the same or substantially the same amino acid sequence as the amino acid sequence represented by SEQ ID NO: 2, or a salt thereof, which comprises using the receptor protein or a salt thereof;

[57] A method of confirming that a central or peripheral neural function-regulating drug or a preventive/therapeutic drug for anxiety, insomnia, excitement, muscular atrophy, anesthesia sensitivity, convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy is an agonist to a G protein-coupled receptor protein containing the same or substantially the same amino acid sequence as the amino acid sequence represented by SEQ ID NO: 2, or a salt thereof, which comprises using the receptor protein or a salt thereof;

[58] A method of confirming that a central or peripheral neural function-regulating drug or a preventive/therapeutic drug for ataxia, paralysis, depression, delayed awakening, deficits in memory and learning, hypotension, dysuria, hypertrophy of bladder, dyspnea, dependence or sensory paralysis is an antagonist to a G protein-coupled receptor protein containing the same or substantially the same amino acid sequence as the amino acid sequence represented by SEQ ID NO: 2, or a salt thereof, which comprises using the receptor protein or a salt thereof;

[59] The confirming method according to [56] through [58], wherein the binding amount of each drug to the receptor protein, its partial peptide, or a salt thereof is determined when each drug is brought in contact with the receptor protein, its partial peptide, or a salt thereof; and the like.

## BRIEF DESCRIPTION OF THE DRAWINGS

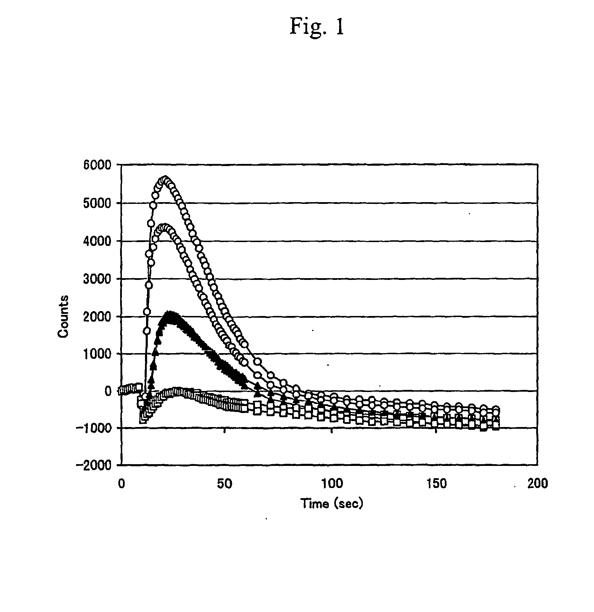

FIG 1 shows the results of changes in intracellular $Ca^{2+}$ level examined when β-alanine is added to CHO-K1 cells expressing TGR7. Counts on the ordinate and Time (sec) on the abscissa indicate, respectively, fluorescence intensity showing the intracellular $Ca^{2+}$ level and passage of time (second) after initiation of the measurement. Open circle, closed triangle and open square denote $10^{-4}$ M, $10^{-5}$ M, and $10^{-6}$ M, respectively.

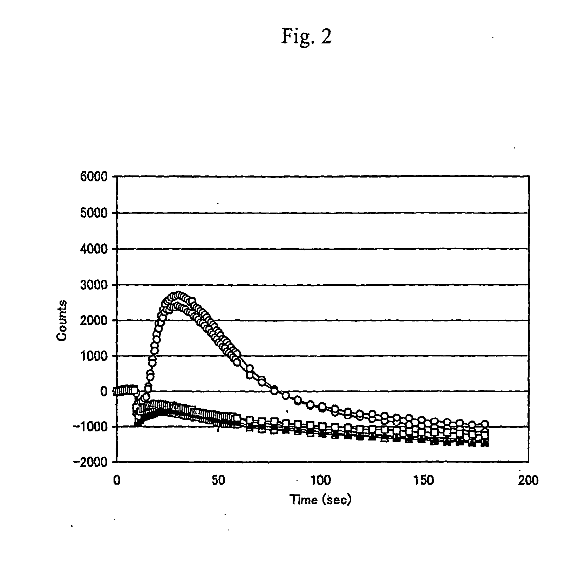

FIG. 2 shows the results of changes in intracellular $Ca^{2+}$ level examined when β-alanine is added to CHO-K1 cells expressing rCB7T084. Counts on the ordinate and Time (sec) on the abscissa indicate, respectively, fluorescence intensity showing the intracellular $Ca^{2+}$ level and passage of time (second) after initiation of the measurement. Open circle, closed triangle and open square denote $10^{-4}$ M, $10^{-5}$ M, and $10^{-6}$ M, respectively.

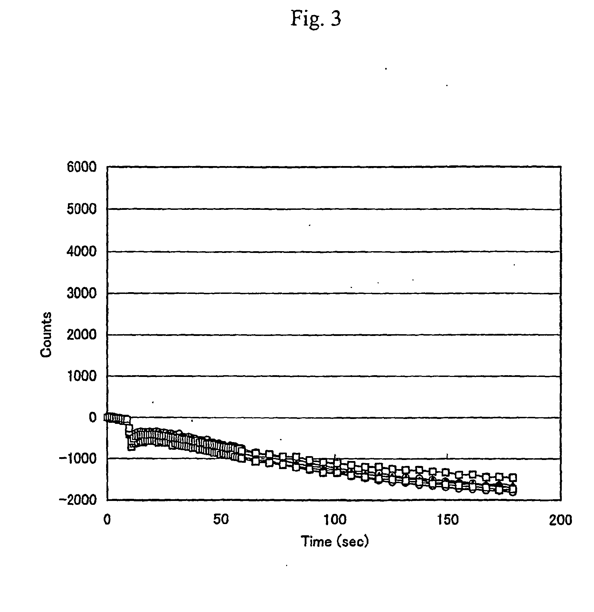

FIG 3 shows the results of changes in intracellular $Ca^{2+}$ level examined when β-alanine is added to CHO-K1 cells expressing an expression vector alone (no insert). Counts on the ordinate and Time (sec) on the abscissa indicate, respectively, fluorescence intensity showing the intracellular $Ca^{2+}$ level and passage of time (second) after initiation of the measurement. Open circle, closed triangle and open square denote $10^{-4}$ M, $10^{-5}$ M, and $10^{-6}$ M, respectively.

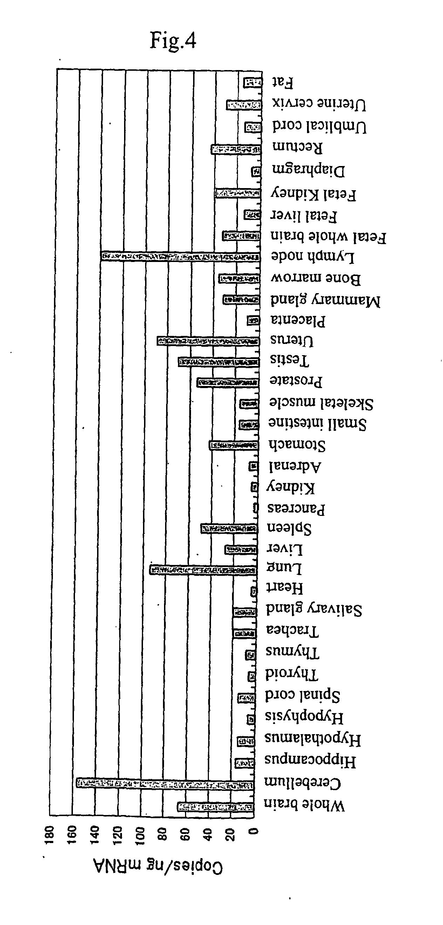

FIG. 4 shows tissue distribution map in human of TGR7mRNA by RT-PCR, wherein copies/ng mRNA denotes the number of copies per ng mRNA.

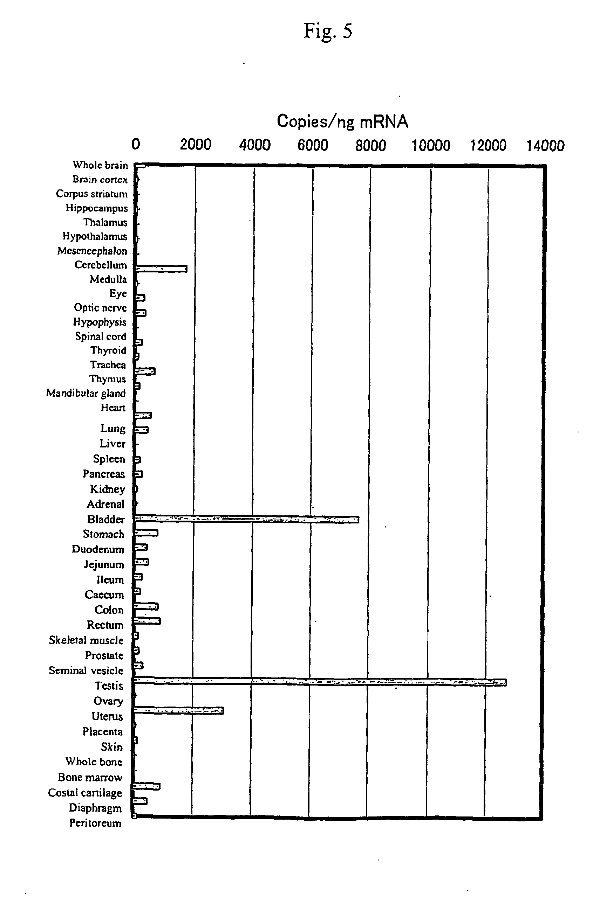

FIG. 5 shows the tissue distribution map in rat of rCB7T084mRNA by RT-PCR, wherein copies/ng mRNA denotes the number of copies per ng mRNA.

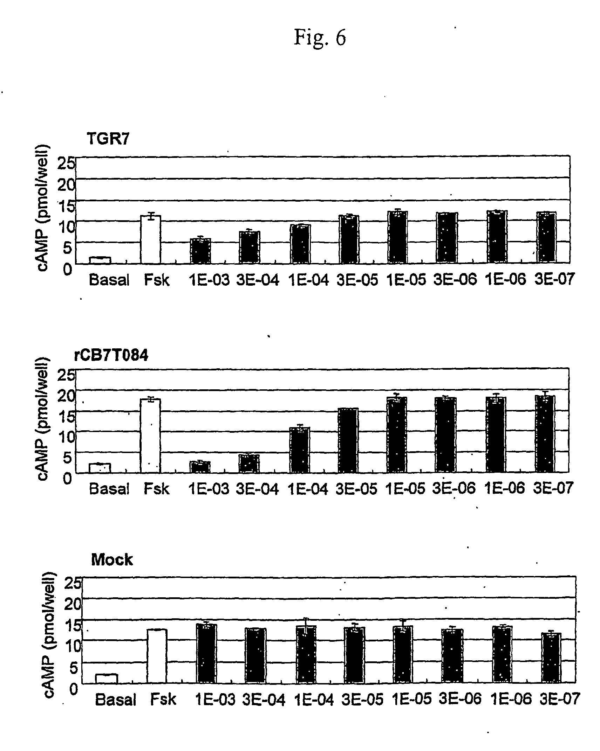

FIG. 6 shows the intracellular cAMP level suppression activity by β-alanine in TGR7- and rCB7T084-expressed CHO cells examined after the level was increased by addition of forskolin. TGR7, rCB7T084 and Mock denote the results obtained by using TGR7-expressed CHO cells, rCB7T084-expressed CHO cells and CHO cells wherein a vector alone was expressed, respectively. Basal denotes the condition without any forskolin stimulation. Fsk denotes the case added with 1 μM of forskolin. The abscissa designates the level of β-alanine added and, 1E-03, 3E-04, 1E-04, 3E-05, 1E-05, 3E-06, 1E-06 and 3E-07 designate $1.0 \times 10^{-3}$ M, $3.0 \times 10^{-4}$ M, $1.0 \times 10^{-4}$ M, $3.0 \times 10^{-5}$ M, $1.0 \times 10^{-5}$ M, $3.0 \times 10^{-6}$ M, $11.0 \times 10^{-6}$ M and $3.0 \times 10^{-7}$ M, respectively. The ordinate designates the intercellular cAMP level.

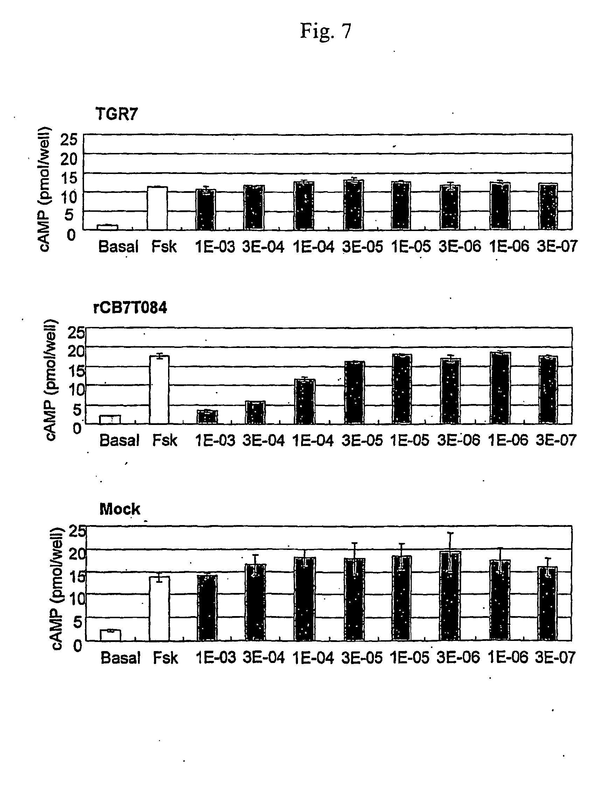

FIG. 7 shows the intracellular cAMP level suppression activity by L-carnosine in TGR7- and rCB7T084-expressed CHO cells examined after the level was increased by addition of forskolin. TGR7, rCB7T084 and Mock denote the results obtained by using TGR7-expressed CHO cells, rCB7T084-expressed CHO cells and CHO cells wherein a vector alone was expressed, respectively. Basal denotes the condition without any forskolin stimulation. Fsk denotes the case added with 1 μM of forskolin. The abscissa designates the level of L-carnosine added and, 1E-03, 3E-04, 1E-04, 3E-05, 1E-05, 3E-06, 1E-06 and 3E-07 designate $3.0 \times 10^{-7}$ M, $1.0 \times 10^{-3}$ M, $3.0 \times 10^{-4}$ M, $1.0 \times 10^{-4}$ M, $3.0 \times 10^{-5}$ M, $1.0 \times 10^{-5}$ M, $3.0 \times 10^{-6}$ M, $1.0 \times 10^{-6}$ M and $3.0 \times 10^{-7}$ M, respectively. The ordinate designates the intercellular cAMP level.

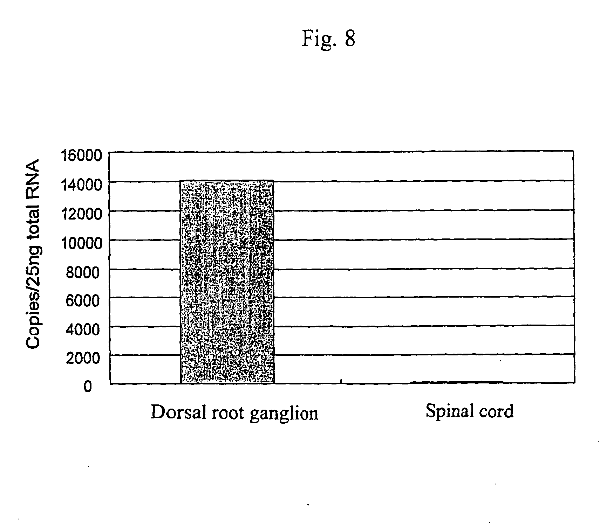

FIG. 8 shows the expression level of rCB7T084mRNA in rat dorsal root ganglion and spinal cord by RT-PCR. Copies/25 ng total RNA denotes the number of copies per 25 ng of total RNA.

BEST MODE FOR CARRYING OUT THE INVENTION

The G protein-coupled receptor protein used in the present invention is a receptor protein containing the same or substantially the same amino acid sequence as the amino acid sequence represented by SEQ ID NO: 2 or SEQ ID NO: 4.

The GPCR of the invention may be any protein derived from any cell of human and mammals (e.g., guinea pig, rat, mouse, rabbit, swine, sheep, bovine, monkey, etc.); any cell (e.g., splenocyte, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells; or the corresponding precursor cells, stem cells, cancer cells, etc.) or hematocyte type cells; or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testicle, testis, ovary, placenta, uterus, bone, joint, skeletal muscle, etc.; the proteins may also be synthetic proteins.

The amino acid sequence which has substantially the same amino acid sequence as that represented by SEQ ID NO: 2 or SEQ ID NO: 4 includes an amino acid sequence having at least about 85% homology, preferably at least about 90% homology and more preferably at least about 95% homology, to the amino acid sequence represented by SEQ ID NO: 2 or SEQ ID NO: 4; etc.

Examples of the protein which contains substantially the same amino acid sequence as that shown by SEQ ID NO: 2 or SEQ ID NO: 4 preferably include a protein having substantially the same amino acid sequence as that shown by SEQ ID NO: 2 or SEQ ID NO: 4 and having the activity substantially equivalent to the amino acid sequence represented by SEQ ID NO: 2 or SEQ ID NO: 4, etc.

Examples of the substantially equivalent activity described above include a ligand binding activity, a signal transduction activity, etc. The term substantially equivalent is used to mean that the activities are the same in nature. Therefore, although it is preferred that activities such as the ligand binding and signal transduction activities, etc. be equivalent (e.g., about 0.01- to about 100-fold, preferably about 0.5- to about 20-fold, more preferably about 0.5- to about 2-fold), quantitative factors such as a level of these activities, a molecular weight of the protein, etc. may differ.

The activities such as ligand binding and signal transduction activities or the like can be determined according to publicly known methods with some modifications thereof, for example, by the ligand determination methods or the screening methods that will be later described.

Also, proteins containing the following amino acid sequences are used as the GPCR of the invention: a) amino acid sequences wherein at least 1 or 2 amino acids (preferably approximately 1 to 30 amino acids, more preferably approximately 1 to 10 amino acids, most preferably several (1 to 5) amino acids) are deleted of the amino acid sequence represented by SEQ ID NO: 2 or SEQ ID NO: 4; b) amino acid sequences wherein at least 1 or 2 amino acids (preferably approximately 1 to 30 amino acids, more preferably approximately 1 to 10 amino acids, and most preferably several (1 to 5) amino acids) are added to the amino acid sequence represented by SEQ ID NO: 2 or SEQ ID NO: 4; c) amino acid sequences wherein at least 1 or 2 amino acids (preferably approximately 1 to 30 amino acids, more preferably approximately 1 to 10 amino acids, and most preferably several (1 to 5) amino acids) in the amino acid sequence represented by SEQ ID NO: 2 or SEQ ID NO: 4 are substituted by other amino acids; or d) combination of these amino acid sequences described above; and the like.

Throughout the present specification, the GPCR of the invention is represented in accordance with the conventional way of describing peptides, that is, the N-terminus (amino terminus) at the left hand and the C-terminus (carboxyl terminus) at the right hand. In the GPCR of the invention including rCB7T084 containing the amino acid sequence shown by SEQ ID NO: 2, the C-terminus may be in any form of a carboxyl group (-COOH), carboxylate (-COO$^-$), an amide (-CONH$_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$-alkyl group, e.g., benzyl, phenethyl, etc., or an α-naphthyl-$C_{1-2}$-alkyl group such as α-naphthylmethyl, etc.; and the like. In addition, pivaloyloxymethyl or the like, which is used widely as an ester for oral administration, may also be used.

Where the GPCR of the invention contains a carboxyl group (or a carboxylate) at a position other than the C-terminus, it may be amidated or esterified and such an amide or ester is also included within the GPCR of the invention. The ester group may be the same group as that described with respect to the C-terminus described above.

Furthermore, examples of the GPCR of the invention include variants of the above proteins, wherein the amino group at the N-terminal methionine residue of the protein supra is protected with a protecting group (for example, a $C_{1-6}$ acyl group such as a $C_{2-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins bound to sugar chains.

As specific examples of the GPCR of the invention, there are employed, for example, rat-derived rCB7T084 consisting of the amino acid sequence represented by SEQ ID NO: 2, human-derived TGR7 consisting of the amino acid sequence represented by SEQ ID NO: 4, and the like. They are publicly known proteins, the former being described in Japanese Patent Laid-Open Application No. 2000-175691 and the latter in WO 01/66750, WO 01/48188, WO 01/36471, WO 01/57085 and WO 01/70814.

As partial peptides of the GPCR of the invention (hereinafter sometimes referred to as the partial peptide), any partial peptide can be used so long as it is a peptide having a part of the amino acid sequence of the GPCR of the invention described above. For example, among protein molecules of the GPCR of the invention, those having a site exposed to the outside of a cell membrane and having substantially the same receptor binding activity as in the GPCR of the invention can be used.

Specifically, the partial peptide of the GPCR of the invention having the amino acid sequence represented by SEQ ID NO: 2 or SEQ ID NO: 4 is a peptide containing the parts analyzed to be extracellular domains (hydrophilic domains) in the hydrophobic plotting analysis. A peptide containing the hydrophobic domain in part can be used as well. In addition, the peptide may contain each domain separately or plural domains together.

In the partial peptides of the present invention, preferred are peptides having at least 20, preferably at least 50, and more preferably at least 100 amino acids, in the amino acid sequence which constitutes the receptor protein of the present invention described above.

The amino acid sequence having substantially the same amino acid sequence includes an amino acid sequence having at least about 85% homology, preferably at least about 90% homology and more preferably at least about 95% homology, to these amino acid sequences.

Herein the term "substantially the same receptor binding activity" has the same significance as described above. The "substantially the same receptor binding activity" can be assayed by the same manner as described above.

In the amino acid sequence described above, the partial peptide of the present invention may contain amino acid sequences, of which at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added;

or, in which at least 1 or 2 amino acids (preferably approximately 1 to 10 amino acids, more preferably several and most preferably approximately 1 to 5 amino acids) are substituted by other amino acids.

In the partial peptide of the present invention, the C-terminus may be in any form of a carboxyl group (-COOH), carboxylate (-COO'), an amide (-CONH$_2$) or an ester (-COOR). Where the partial peptide of the present invention contains a carboxyl group (or a carboxylate) at a position other than the C-terminus, it may be amidated or esterified and such an amide or ester is also included within the partial peptide of the present invention. In this case, the ester group may be the same group as that described with respect to the C-terminus described above.

As in the GPCR of the invention described above, the partial peptide of the present invention further includes those in which the amino group of the amino acid residue of the N-terminal methionine residue is protected by a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycopeptides, to which sugar chains are bound, and the like.

For salts of the GPCR of the invention or its partial peptide, preferred are physiologically acceptable salts with acids or bases, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The GPCR of the invention or salts thereof may be manufactured by a publicly known method used to purify receptor proteins from human or mammalian cells or tissues described above, or by culturing a transformant that contains the DNA encoding the GPCR of the invention, as will be later described. Furthermore, the GPCR of the invention or its salts may also be manufactured by the methods for synthesizing proteins or by modifications thereof, which will also be described hereinafter.

Where the GPCR of the invention or its salts are manufactured from human or mammalian tissues or cells, human or mammalian tissues or cells are homogenized, then extracted with an acid or the like, and the extract is isolated and purified by a combination of chromatography techniques such as reverse phase chromatography, ion exchange chromatography, and the like.

To synthesize the GPCR of the invention or its partial peptides, or salts or amides thereof according to the present invention, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which $\alpha$-amino groups and functional groups on the side chains are appropriately protected are condensed on the resin in the order of the sequence of the objective protein according to various condensation methods publicly known in the art. At the end of the reaction, the protein is cut out from the resin and at the same time, the protecting groups are removed. Then, intramolecular disulfide bond-forming reaction is performed in a highly diluted solution to obtain the objective protein or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20 to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups used in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids, in which the amino groups are activated in the starting material, for example, the corresponding phosphoric amides are employed.

To eliminate (split off) the protecting groups, there are used catalytic reduction under hydrogen gas flow in the presence of a catalyst such as Pd-black, Pd-carbon, etc.; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine, piperazine, etc.; and reduction with sodium in liquid ammonia. The elimination of the protecting group by the acid treatment described above is carried out generally at a temperature of approximately -20 to 40°C. In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of the functional groups that should not be involved in the reaction of the starting materials, protecting groups, elimination of the protecting groups, activation of functional groups involved in the reaction, or the like may be appropriately selected from publicly known groups and publicly known means.

In another method for obtaining the amides of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide or its salts of the GPCR of the invention can be manufactured by publicly known methods for peptide synthesis, or by cleaving the GPCR of the invention with an appropriate peptidase. For the methods for peptide synthesis, for example, either solid phase synthesis or liquid phase synthesis may be used. That is, the partial peptide or amino acids that can construct the GPCR of the invention are condensed with the remaining part. Where the product contains protecting groups, these protecting groups are removed to give the desired peptide. Publicly known methods for condensation and elimination of the protecting groups are described in a) - e) below.

a) M. Bodanszky & M.A. Ondetti: Peptide Synthesis, Interscience Publishers, New York (1966)

b) Schroeder & Luebke: The Peptide, Academic Press, New York (1965)

c) Nobuo Izumiya, et al.: *Peptide Gosei-no-Kiso to Jikken* (Basics and experiments of peptide synthesis), published by Maruzen Co. (1975)

d) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)

e) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the methods above is in a free form, the peptide can be converted into an appropriate salt by a publicly known method; conversely when the peptide is obtained in a salt form, it can be converted into a free form by a publicly known method.

The polynucleotide encoding the GPCR of the invention may be any polynucleotide so long as it contains the base sequence (DNA or RNA, preferably DNA) encoding the GPCR of the invention described above. Such a polynucleotide may also be any one of DNA, or RNA such as mRNA, etc. encoding the GPCR of the invention, and may be double-stranded or single-stranded. Where the polynucleotide is double-stranded, it may be double-stranded DNA, double-stranded RNA or DNA:RNA hybrid. Where the polynucleotide is single-stranded, it may be a sense strand (i. e., a coding strand) or an antisense strand (i.e., a non-coding strand).

Using the polynucleotide encoding the GPCR of the invention, mRNA of the GPCR of the invention can be quantified by, for example, the publicly known method published in separate volume of *Jikken Igaku* 15 (7) "New PCR and its application" (1997), or by its modifications.

The DNA encoding the GPCR of the invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the GPCR of the invention may be any DNA, so long as it is, for example, a DNA containing the base sequence represented by SEQ ID NO: 1 or SEQ ID NO: 3, or any DNA having a base sequence hybridizable to the base sequence represented by SEQ ID NO: 1 or SEQ ID NO: 3 under high stringent conditions and encoding a receptor protein which has the activities substantially equivalent to those of the GPCR of the invention (e.g., the ligand-binding activities, the signal transduction activities, etc.).

Examples of the DNA hybridizable to the DNA containing the base sequence represented by SEQ ID NO: 1 or SEQ ID NO: 3 under highly stringent conditions include a DNA containing a base sequence having at least about 85% homology, preferably at least about 90% homology and more preferably at least about 95% homology, to the base sequence represented by SEQ ID NO: 1 or SEQ ID NO: 3.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 to 40 mM, preferably about 19 to 20 mM at a temperature of about 50 to 70°C, preferably about 60 to 65°C. In particular, hybridization conditions in a sodium concentration of about 19 mM at a temperature of about 65°C are most preferred.

More specifically, as the DNA encoding rat-derived rCB7T084 consisting of the amino acid sequence represented by SEQ ID NO: 2, there may be employed a DNA consisting of the base sequence represented by SEQ ID NO: 1; etc.

As the DNA encoding human-derived TGR7 consisting of the amino acid sequence represented by SEQ ID NO: 4, there may be employed a DNA consisting of the base sequence represented by SEQ ID NO: 1; etc.

The term polynucleotide comprising a part of the base sequence of the DNA encoding the GPCR of the invention or a part of the base sequence complementary to the DNA is used to mean that the polynucleotide embraces not only the DNA encoding the partial peptide of the present invention described below but also RNA.

According to the present invention, antisense polynucleotides (nucleic acids) that can inhibit the replication or expression of GPR genes can be designed and synthesized based on the base sequence information of the cloned or determined DNA encoding the GPCR of the invention. Such a polynucleotide (nucleic acid) is capable of hybridizing to RNA of the GPCR of the invention gene to inhibit the synthesis or function of said RNA or capable of modulating or controlling the expression of the GPCR of the invention gene via interaction with the GPCR of the invention-associated RNA. Polynucleotides complementary to the selected sequences of RNA associated with the GPR of the invention and polynucleotides specifically hybridizable to RNA associated with the GPR of the invention are useful in modulating or controlling the expression of the GPCR of the invention gene in vivo and in vitro, and useful for the treatment or diagnosis of diseases. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the genes for the GPCR of the invention, the 5' end hairpin loop, 5' end 6-base-pair repeats, 5' end untranslated region, polypeptide translation initiation codon, protein coding region, ORF translation initiation codon, 3' end untranslated region, 3' end palindrome region, and 3' end hairpin loop, may be selected as preferred target regions, though any other region may be selected as a target in the genes for the GPCR of the invention.

The relationship between the targeted nucleic acids and the polynucleotides complementary to at least a part of the target, specifically the relationship between the target and the polynucleotides hybridizable to the target, can be denoted to be "antisense." Examples of the antisense polynucleotides include polydeoxyribonucleotide containing 2-deoxy-D-ribose, polyribonucleotide containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. They may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e. g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain changed or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e. g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the G protein-coupled receptor protein in vivo and in vitro. The nucleic acid can be applied to cells by a variety of publicly known methods.

The DNA encoding the partial peptide of the present invention may be any DNA so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example, (1) a DNA containing a partial base sequence of the DNA having the base sequence represented by SEQ ID NO: 1 or SEQ ID NO: 3, or (2) any DNA containing a partial base sequence of the DNA having a DNA hybridizable to the DNA represented by SEQ ID NO: 1 or SEQ ID NO: 3 under highly stringent conditions and encoding the receptor protein which has the activities (e.g., the ligand-biding activities, the signal transduction activities, etc.) substantially equivalent to those of the GPCR of the invention; etc.

Examples of the DNA that is hybridizable to the base sequence represented by SEQ ID NO: 1 or SEQ ID NO: 3 include DNA containing a base sequence having at least about 85% homology, preferably at least about 90% homology and more preferably at least about 95% homology, to the base sequence represented by SEQ ID NO: 1 or SEQ ID NO: 3.

For cloning of the DNA that completely encodes the GPCR of the invention or its partial peptide (hereinafter sometimes collectively referred to as the GPCR of the invention), the DNA may be amplified by PCR using synthetic DNA primers containing a part of the base sequence of the GPCR of the invention, or the DNA inserted into an appropriate vector can be selected by hybridization with a labeled DNA fragment or synthetic DNA that encodes a part or entire region of the GPCR of the invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by publicly known methods such as the ODA-LA PCR method, the Gapped duplex method or the Kunkel method or its modifications by using publicly known kits available as Mutan™-superExpress Km (manufactured by TaKaRa Shuzo Co., Ltd.), Mutan™-K (manufactured by TaKaRa Shuzo Co., Ltd.), etc.

The cloned DNA encoding the GPCR of the invention can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the GPCR of the invention can be manufactured, for example, by (a) excising the desired DNA fragment from the DNA encoding the GPCR of the invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV promoter or SRα promoter is preferably used. Where the host is bacteria of the genus Escherichia, preferred examples of the promoter include trp promoter, lac promoter, recA promoter, λP$_L$ promoter, lpp promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp$^r$), neomycin resistant gene (hereinafter sometimes abbreviated as Neo$^r$, G418 resistance), etc. In particular, when dhfr gene is used as the selection marker in CHO (dhfr$^-$) cells, selection can also be made on thymidine free media.

If necessary, a signal sequence that matches with a host is added to the N-terminus of the receptor protein of the present invention. Examples of the signal sequence that can be used are PhoA signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the GPCR of the invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., $\underline{60}$, 160 (1968)), JM103 (Nucleic Acids Research, $\underline{9}$, 309 (1981)], JA221 [Journal of Molecular Biology, $\underline{120}$, 517 (1978)], HB101 [Journal of Molecular Biology, $\underline{41}$, 459 (1969)), C600 (Genetics, $\underline{39}$, 440 (1954)], etc.

Examples of the bacteria belonging to the genus Bacillus include Bacillus subtilis MI114 [Gene, $\underline{24}$, 255 (1983)], 207-21 [Journal of Biochemistry, $\underline{95}$, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, $\underline{13}$, 213-217 (1977)).

As the insect, for example, a larva of Bombyx mori can be used [Maeda, et al., Nature, $\underline{315}$, 592 (1985)].

Examples of animal cells include monkey cells COS-7, Vero, Chinese hamster cells CHO (hereinafter referred to as CHO cells), dhfr gene deficient Chinese hamster cells CHO (hereinafter briefly referred to as CHO (dhfr⁻) cell), mouse L cells, mouse AtT-20, mouse myeloma cells, rat GH3, human FL cells, human HEK293 cells, etc.

Bacteria belonging to the genus Escherichia can be transformed, for example, by the method described in Proc. Natl. Acad. Sci. U.S.A., $\underline{69}$, 2110 (1972), Gene, $\underline{17}$, 107 (1982), etc.

Bacteria belonging to the genus Bacillus can be transformed, for example, by the method described in Molecular & General Genetics, $\underline{168}$, 111 (1979).

Yeast can be transformed, for example, by the method described in Methods in Enzymology, $\underline{194}$, 182-187 (1991), Proc. Natl. Acad. Sci. U.S.A., $\underline{75}$, 1929 (1978), etc.

Insect cells or insects can be transformed, for example, according to the method described in Bio/Technology, $\underline{6}$, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995) (published by Shujunsha), or Virology, $\underline{52}$, 456 (1973).

Thus, the transformant transformed with the expression vector containing the DNA encoding the GPCR of the invention can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids (Miller, Journal of Experiments in Molecular Genetics, 431-433, Cold Spring Harbor Laboratory, New York, 1972). If necessary, a chemical such as 3β-indolylacrylic acid can be added to the medium thereby to activate the promoter efficiently.

Where the bacteria belonging to the genus Escherichia are used as the host, the transformant is usually cultivated at about 15 to 43°C for about 3 to 24 hours. If necessary, the culture may be aerated or agitated.

Where the bacteria belonging to the genus Bacillus are used as the host, the transformant is cultivated generally at about 30 to 40°C for about 6 to 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., $\underline{77}$, 4505 (1980)] or in SD medium supplemented with 0.5% Casamino acids [Bitter, G. A. et al., Proc. Natl. Acad. Sci. U.S.A., $\underline{81}$, 5330 (1984)]. Preferably, pH of the medium is adjusted to about 5 to about 8. In general, the transformant is cultivated at about 20°C to about 35°C for about 24 to 72 hours. If necessary, the culture can be aerated or agitated.

Where insect cells or insects are used as the host, the transformant is cultivated in, for example, Grace's Insect Medium (Grace, T. C. C., Nature, $\underline{195}$, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the GPCR of the invention can be produced into the cell, in the cell membrane or out of the cell of the transformant.

The GPCR of the invention can be separated and purified from the culture described above by the following procedures.

When the GPCR of the invention is extracted from the culture or cells, after cultivation the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the GPCR of the invention can be obtained. The buffer used for the procedures may contain a protein modifier such as urea or guanidine hydrochloride, or a surfactant such as Triton X-100™, etc. When the GPCR of the invention is secreted in the culture, after completion of the cultivation the supernatant can be separated from the transformants or cells to collect the supernatant by a publicly known method.

The GPCR of the invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

In the case that the GPCR of the invention thus obtained is in a free form, it can be converted into the salt by publicly known methods or modifications thereof. On the other hand, when the GPCR of the invention is obtained in the form of a salt, it can be converted into the free form or in the form of a different salt by publicly known methods or modifications thereof.

The GPCR of the invention produced by the recombinant can be treated, prior to or after the purification, with an appropriate protein modifying enzyme so that the GPCR of the invention can be appropriately modified to partially remove a polypeptide. Examples of the protein-modifying enzyme include trypsin, chymotrypsin, arginyl endopeptidase, protein kinase, glycosidase or the like.

The activity of the thus produced the GPCR of the invention or salts thereof can be determined by a binding experiment to a labeled ligand, by an enzyme immunoassay using a specific antibody, or the like.

Antibodies to the GPCR of the invention may be any of polyclonal antibodies and monoclonal antibodies, as long as they are capable of recognizing the GPCR of the invention.

The antibodies to the GPCR of the invention may be manufactured by publicly known methods for manufacturing antibodies or antisera, using as antigens the GPCR of the invention.

[Preparation of monoclonal antibody]

(a) Preparation of monoclonal antibody-producing cells

The GPCR of the invention is administered to mammals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the receptor protein, which will be described later, with the antiserum followed by assaying the binding activity of the labeling agent bound to the antibody. The fusion may be operated, for example, by the known Koehler and Milstein method [Nature, 256, 495 (1975)]. Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A

**EP 1 491 207 A1**

preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with an antigen of the receptor protein directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the receptor protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be manufactured by publicly known methods or modifications thereof. For example, a complex of immunogen (the GPCR of the invention as an antigen) and a carrier protein is prepared, and a mammal is immunized with the complex in a manner similar to the method described above for the manufacture of monoclonal antibodies. The product containing the antibody to the GPCR of the invention is collected from the immunized animal followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

The ligand to the GPCR of the invention is β-alanine or L-carnosine. L-Carnosine is composed of β-alanine and histidine bound thereto. β-Alanine is structurally akin to glycine or GABA, which is an inhibitory neurotransmitter.

Also, the GPCR of the invention is expressed specifically in the cerebellum, dorsal root ganglion, bladder, testis, uterus, etc. It is thus considered that the GPCR of the invention will take part in regulating neurotransmission in the central or peripheral nervous system.

Therefore, the DNA encoding the GPCR of the invention (hereinafter sometimes referred to briefly as the DNA of the present invention), the antibody to the GPCR of the invention (hereinafter sometimes referred to briefly as the antibody of the present invention) and the antisense DNA to the DNA of the present invention (hereinafter sometimes merely referred to as the antisense DNA of the present invention) have the following applications.

(1) Preventive/therapeutic agent for diseases associated with dysfunction of the GPCR of the invention

It is considered that the GPCR of the invention would be engaged in the central and/or peripheral neural functions. Accordingly, a) the GPCR of the invention or b) the DNA encoding the GPCR of the invention can be used as a preventive/therapeutic agent, etc. for diseases associated with dysfunction of the GPCR of the invention, especially abnormalities in the central and/or peripheral neural functions.

For example, when the physiological activities of β-alanine or L-carnosine, which is the ligand, cannot be expected in a patient (deficiency of the GPCR of the invention) due to a decrease of the GPCR of the invention in the body, the amount of the GPCR of the invention can be increased in the boy of the patient a) by administering the GPCR of the invention to the patient thereby to supplement the amount of the GPCR of the invention; or b) (i) by administering the DNA encoding the GPCR of the invention to the patient and expressing the same, or (ii) by inserting and expressing the DNA encoding the GPCR of the invention in the objective cells and then transplanting the cells to the patient, thus increasing the amount of the GPCR of the invention in the body of the patient, whereby the activities of the ligand can be sufficiently exhibited. That is, the DNA encoding the GPCR of the invention is useful as a safe and low toxic preventive/therapeutic agent for diseases associated with dysfunction of the GPCR of the invention, especially diseases associated with abnormalities in central and/or peripheral neural functions.

Specifically, the GPCR of the invention or the DNA of the present invention can be used as a preventive/therapeutic drug for diseases associated with abnormalities in central neural functions, e.g., an antianxiety drug, a hypnotic sedative, a muscle relaxant, an anesthetic fortifier, an anticonvulsant, an anti-dependence drug, an anti-schizophrenic drug, an anti-epilepsy drug, an anti-psychosomatic drug, etc.; or as a preventive/therapeutic drug for diseases associated with abnormalities in peripheral neural functions, e.g., incontinence, neurogenic hypertension, threatened abortion, threatened premature delivery, male infertility, autonomic imbalance, etc. Moreover, the GPCR of the invention or the DNA of the present invention can be used as a preventive/therapeutic drug for diseases such as cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia or palsy, etc.

Also, β-alanine or L-carnosine can be used as a pharmaceutical including an enhancer of signal transduction activity of the GPCR of the invention, a central and/or peripheral neural function-regulating drug, etc., a preventive and/or therapeutic drug for diseases associated with dysfunction of the GPCR of the invention, especially diseases associated with abnormalities in central neural functions. Specifically, β-alanine or L-carnosine can be used as a preventive/therapeutic drug for diseases associated with abnormalities in central neural functions, e.g., an antianxiety drug, a hypnotic sedative, a muscle relaxant, an anesthetic fortifier, an anticonvulsant, an anti-dependence drug, an anti-schizophrenic drug, an anti-epilepsy drug, an anti-psychosomatic drug, etc.; or as a preventive/therapeutic drug for diseases associated with abnormalities in peripheral neural functions, e.g., incontinence, neurogenic hypertension, threatened abortion, threatened premature delivery, male infertility, autonomic imbalance, etc. Moreover, β-alanine or L-carnosine can be used as a preventive/therapeutic drug for diseases such as cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia or palsy, etc.

When the GPCR of the invention is used as the pharmaceuticals described above, the GPCR of the invention can be prepared into a pharmaceutical composition in a conventional manner.

On the other hand, where the DNA of the present invention is used as t the pharmaceuticals described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, a) the GPCR of the invention or b) the DNA of the present invention can be used orally, for example, in the form of tablets which may be sugar coated if necessary, capsules, elixirs, microcapsules etc., or parenterally in the form of injectable preparations such as a sterile solution and a suspension in water or with other pharmaceutically acceptable liquid. These preparations can be manufactured by mixing a) the GPCR of the invention or b) the DNA of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate, benzyl alcohol, etc.

The pharmaceuticals described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the GPCR of the invention varies depending on subject to be administered, organs to be administered, conditions, methods for administration, etc.; in oral administration, e.g., for the patient with schizophrenia, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, methods for administration, etc. but it is advantageous, e.g., for the patient with schizophrenia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The dose of the DNA of the present invention varies depending on subject to be administered, organs to be administered, conditions, methods for administration, etc.; in oral administration, e.g., for the patient with schizophrenia, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, methods for administration, etc. but it is advantageous, e.g., for the patient with schizophrenia, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.


(2) Gene diagnostic agent

By using the DNA and antisense DNA of the present invention as probes, abnormalities (gene abnormalities) of the DNA or mRNA encoding the GPCR of the invention or its partial peptides in human or mammals (e.g., rats, mice, rabbits, sheep, swine, bovine, cats, dogs, monkeys, etc.) can be detected, and the DNA and antisense DNA are thus useful as gene diagnostic agents for the damage against the DNA or mRNA, its mutation, or its reduced expression, or increased expression or overexpression of the DNA or mRNA.

The gene diagnosis described above using the DNA or antisense DNA can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc..

Where a reduced expression of the GPCR of the invention is detected, e.g., by northern hybridization, it can be diagnosed that one suffers from, for example, diseases associated with dysfunction of the GPCR of the invention, especially diseases associated with abnormalities in central and/or peripheral neural functions, or it is highly likely to suffer from these disease in the future.

Also, where the overexpression of the GPCR of the invention is detected, e.g., by northern hybridization, it can be diagnosed that one suffers from, for example, diseases caused by overexpression of the GPCR of the invention, especially diseases associated with abnormalities in central and/or peripheral neural functions, or it is highly likely to suffer from these disease in the future.

The diseases associated with dysfunction of the GPCR of the invention are, for example, diseases with abnormalities in central neural functions including anxiety, insomnia, excitement, muscular atrophy, anesthesia resistance, convulsion, dependence, schizophrenia, epilepsy, psychosomatic disorders, etc.; or diseases with abnormalities in peripheral neural functions including incontinence, neurogenic hypertension, threatened abortion, threatened premature delivery, male infertility, autonomic imbalance, etc.; furthermore, cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia or palsy, etc.

The diseases caused by overexpression of the GPCR of the invention include, for example, ataxia, paralysis, depression, delayed awakening, deficits in memory and learning, hypotension, dysuria, hypertrophy of bladder, dyspnea, dependence or sensory paralysis, etc.

(3) Pharmaceutical comprising the compound that changes the expression level of the GPCR of the invention

By using the DNA of the present invention as a probe, the DNA can be used for screening the compound that changes an expression level of the GPCR of the invention.

That is, the present invention provides a method of screening the compound that changes the expression level of the GPCR of the invention, which comprises measuring the amount of mRNA in the GPCR of the invention contained, for example, in (i) a) blood, b) particular organs, c) tissues or cells isolated from the organs of non-human mammals or in (ii) transformants, etc.

The amount of mRNA in the GPCR of the invention can be specifically measured as follows.

(i) Normal or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., anti-dementia agents, hypotensive agents, anticancer agents, antiobestic agents, etc.) or physical stress (e.g., soaking stress, electric shock, light and darkness, low temperature, etc.), and the blood, particular organs (e.g., brain, liver, kidney, etc.), or tissues or cells isolated from the organs are obtained after a specified period of time.

The mRNA of the GPCR of the invention contained in the thus obtained cells is extracted from the cells, for example, in a conventional manner and quantified by means of, e.g., TaqManPCR, or may also be analyzed by northern blot technique by publicly known methods.

(ii) Transformants that express the GPCR of the invention are prepared according to the methods described above, and the mRNA encoding the GPCR of the invention can be quantified and analyzed, as described above.

The compound that changes the expression level of the GPCR of the invention can be screened by the following procedures.

(i) To normal or disease models of non-human mammals, a test compound is administered at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of mRNA encoding the GPCR of the invention contained in cells are quantified and analyzed.

(ii) Transformants are cultured in a conventional manner and a test compound is mixed in the culture medium. After a specified time (after 1 day to 7 days, preferably after 1 day to 3 days, more preferably after 2 to 3 days), the amount of mRNA encoding the GPCR of the invention contained in the transformants can be quantified and analyzed.

The compound or its salt, which is obtained by the screening methods of the present invention, is the compound that changes the expression level of the GPCR of the invention. Specifically, it is (a) the compound that potentiates the cell stimulating activities mediated by the GPCR of the invention (e.g., the activities that promote or suppress arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) by increasing the expression level of the GPCR of the invention; and (b) the compound that attenuates the cell-stimulating activities by decreasing the expression level of the GPCR of the invention.

The compounds include peptides, proteins, non-peptide compounds, synthetic compounds, fermentation products, etc. They may be novel or known compounds.

The ligand to the GPCR of the invention is β-alanine or L-carnosine as described above. Accordingly, the compound that changes the expression level of the GPCR of the invention obtained by the screening methods described above can be used as a central and/or peripheral neural function-regulating agent or as a preventive/therapeutic agent for diseases associated with dysfunction of the GPCR of the invention, especially abnormalities in central and/or peripheral neural functions.

Specifically, the compound that increases the expression level of the GPCR of the invention to enhance the cell stimulating activities is useful as a safe and low toxic preventive/therapeutic agent for diseases associated with dysfunction of the GPCR of the invention.

The compound that decreases the expression level of the GPCR of the invention to attenuate the cell stimulating activities is useful as a safe and low toxic preventive/therapeutic agent for diseases caused by overexpression of the GPCR of the invention.

The diseases associated with dysfunction of the GPCR of the invention include diseases with abnormalities in central neural functions including anxiety, insomnia, excitement, muscular atrophy, anesthesia resistance, convulsion, dependence, schizophrenia, epilepsy, psychosomatic disorders, etc.; or diseases with abnormalities in peripheral neural functions including incontinence, neurogenic hypertension, threatened abortion, threatened premature delivery, male infertility, autonomic imbalance, etc.; furthermore, cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia, palsy, etc.

The diseases caused by overexpression of the GPCR of the invention include ataxia, paralysis, depression, delayed awakening, deficits in memory and learning, hypotension, dysuria, hypertrophy of bladder, dyspnea, dependence or sensory paralysis, etc.

Where the compound or its salt, which is obtained by the screening methods of the present invention, is used as a pharmaceutical composition, the compound or its salt can be formed into a pharmaceutical preparation in a conventional manner.

For example, the compound can be used orally in the form of tablets which may be tablets, if necessary, coated with sugar, capsules, elixirs, microcapsules, etc., or parenterally in the form of injectable preparations such as a sterile solution or a suspension in water or with other pharmaceutically acceptable liquid. These preparations can be manufactured, e.g., by mixing the compound, with a physiologically acceptable known carrier, flavoring agent, excipient, vehicle, antiseptic, stabilizer, binder, etc., in a unit dosage form required in a generally accepted manner applied to making pharmaceutical preparations. The active ingredient in the preparation is controlled in such an amount that an appropriate dose is obtained within the specified range given.

Additives miscible with tablets, capsules, etc. include a binder such as gelatin, corn starch, tragacanth or gum arabic, an excipient such as crystalline cellulose, a swelling agent such as corn starch, gelatin, alginic acid, etc., a lubricant such as magnesium stearate, a sweetening agent such as sucrose, lactose or saccharin, a flavoring agent such as peppermint, akamono oil or cherry, and the like. When the unit dosage is in the form of capsules, liquid carriers such as oils and fats may further be used together with the additives described above. A sterile composition for injection may be formulated following a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline, an isotonic solution containing glucose and other auxiliary agents (e.g., D-sorbitol, D-mannitol, sodium chloride, etc.) or the like, which may be used in combination with an appropriate dissolution aid such as an alcohol (e.g., ethanol), a polyalcohol (e.g., propylene glycol, polyethylene glycol), a nonionic surfactant (e.g., polysorbate 80™ and HCO-50), etc. As an oily medium, for example, sesame oil, soybean oil or the like may be used, which can be used in combination with a dissolution aid such as benzyl benzoate, benzyl alcohol, etc.

Furthermore, the pharmaceutical drug described above may also be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the compound or its salts varies depending on subject to be administered, target organ, conditions, methods for administration, etc.; in oral administration, the dose for the patient with schizophrenia (as 60 kg body weight) is normally about 0.1 mg to about 100 mg, preferably about 1.0 to about 50 mg, and more preferably about 1.0 to about 20 mg per day. In parenteral administration, the single dose may vary depending on subject to be administered, target organ, conditions, methods for administration, etc. but it is advantageous to administer the active ingredient intravenously to the patient with schizophrenia (as 60 kg body weight) in a daily dose of about 0.01 to about 30 mg, preferably about 0.1 to about 20 mg, and more preferably about 0.1 to about 10 mg. For other animal species, the corresponding dose as converted per 60 kg body weight can be administered.

(4) Quantification of the GPCR of the invention and a method for diagnosis

The antibodies of the present invention are capable of specifically recognizing the GPCR of the invention. Therefore, the antibodies can be used to quantify the GPCR of the invention in a test fluid, especially for quantification by the sandwich immunoassay, etc.

That is, the present invention provides, for example, the following quantification methods:

(i) a method for quantification of the GPCR of the invention in a test fluid, which comprises competitively reacting the antibody of the present invention with the test fluid and a labeled form of the GPCR of the invention, and measuring a ratio of the labeled GPCR of the invention bound to the antibody; and,

(ii) a method for quantification of the GPCR of the invention in a test fluid, which comprises reacting the test fluid with the antibody of the present invention immobilized on a carrier and a labeled form of the antibody of the present invention simultaneously or sequentially, and measuring the activity of the label on the immobilized carrier.

In the quantification method (ii) described above, it is preferred that one antibody recognizes the N-terminal region of the GPCR of the invention, and another antibody reacts with the C-terminal region of the GPCR of the invention.

Using the monoclonal antibodies to the GPCR of the invention, the GPCR of the invention can be assayed. The GPCR of the invention can also be detected by tissue staining or the like. For this purpose, the antibody molecule itself may be used, or $F(ab')_2$, Fab' or Fab fractions of the antibody molecule may also be used.

The quantification methods of the GPCR of the invention using the antibodies are not particularly limited. Any quantification method can be used, so long as the amount of antibody, antigen, or antibody-antigen complex corresponding to the amount of antigen (e.g., the amount of the GPCR of the invention) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, $[^{125}I]$, $[^{131}I]$, $[^{3}H]$, $[^{14}C]$ and the like are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used are fluorescamine and fluorescein isothiocyanate are used. For the luminescent substance, there are used, for example, luminol, luminol derivatives, luciferin, and lucigenin. Furthermore, the biotin-avidin system may be used for binding antibody or antigen to the label.

For immobilization of antigen or antibody, physical adsorption may be used. Chemical binding methods conventionally used for insolubilization or immobilization of the GPCR of the invention, enzymes or the like may also be used. For the carrier, for example, insoluble polysaccharides such as agarose, dextran, cellulose, etc.; synthetic resin such as polystyrene, polyacrylamide, silicon, etc., and glass or the like are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the GPCR of the invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the GPCR of the invention by the sandwich method, antibodies that bind to different sites of the GPCR of the invention are preferably used as the monoclonal antibodies of the present invention for the primary and secondary reactions. That is, in the antibodies used for the primary and secondary reactions are, for example, when the antibody used in the secondary reaction recognizes the C-terminal region of the GPCR of the invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the GPCR of the invention are constructed by adding the usual technical consideration in the art to the conventional conditions and procedures. For the details of these general technical means, reference can be made to the following reviews and texts.

For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the GPCR of the invention can be quantified with high sensitivity, using the antibodies of the present invention.

Where a decreased level of the GPCR of the invention is detected by quantifying the GPCR of the invention level using the antibodies of the present invention, it can be diagnosed that one suffers from, for example, diseases associated with dysfunction of the GPCR of the invention, especially abnormalities in central and/or peripheral neural functions, or it is highly likely to suffer from these disease in the future.

Where an increase level of the GPCR of the invention is detected, it can be diagnosed that one suffers from, for example, diseases caused by overexpression of the GPCR of the invention, or it is highly likely to suffer from these disease in the future.

The diseases associated with dysfunction of the GPCR of the invention include anxiety, insomnia, excitement, muscular atrophy, anesthesia resistance, convulsion, dependence, schizophrenia, epilepsy, psychosomatic disorders, etc.; or diseases with abnormalities in peripheral neural functions including incontinence, neurogenic hypertension, threatened abortion, threatened premature delivery, male infertility, autonomic imbalance, etc.; furthermore, cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia, palsy, etc.

The diseases caused by overexpression of the GPCR of the invention include, for example, ataxia, paralysis, depression, delayed awakening, deficits in memory and learning, hypotension, dysuria, hypertrophy of bladder, dyspnea, dependence or sensory paralysis, etc.

(5) Methods for determination of agonists to the GPCR of the invention

β-Alanine or L-carnosine binds to the GPCR of the invention so that suppression of the intracellular cAMP production is observed. Thus, the GPCR of the invention is useful as a reagent for searching or determining agonists (including naturally occurring ligands) to the GPCR of the invention other than β-alanine or L-carnosine, using the intracellular signal as an indicator.

That is, the present invention provides a method of determining the agonist to the GPCR of the invention, which comprises assaying the intracellular cAMP production suppressing activity mediated by the GPCR of the invention, when a test compound is brought in contact with a cell containing the GPCR of the invention.

Examples of test compounds include publicly known ligands (for example, angiotensin, bombesin, canavinoid, cholecystokinin, glutamine, serotonin, melatonin, neuropeptide Y, opioids, purines, vasopressin, oxytocin, PACAP (e. g., PACAP27, PACAP38), secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CORP (calcitonin gene-related peptide), leukotrienes, pancreastatin, prostaglandins, thromboxane, adenosine, adrenaline, the chemokine superfamily (e.g., the CXC chemokine subfamily such as IL-8, GRO$\alpha$, GRO$\beta$, GRO$\gamma$, NAP-2, ENA-78, GCP-2, PF4, IP-10, Mig, PBSF/SDF-1, etc.; the CC chemokine subfamily such as MCAF/MCP-1, MCP-2, MCP-3, MCP-4, eotaxin, RANTES, MIP-1$\alpha$, MIP-1$\beta$, HCC-1, MIP-3$\alpha$/LARC, MIP-3$\beta$/ELC, I-309, TARC, MIPF-1, MIPF-2/eotaxin-2, MDC, DC-CK1/PARC, SLC, etc.; the C chemokine subfamily such as lymphotactin, etc.; the CX3C chemokine subfamily such as fractalkine, etc.), endothelin, enterogastrin, histamin, neurotensin, TRH, pancreatic polypeptide, galanin, lysophosphatidic acid (LPA), sphingosine 1-phosphate, etc.) as well as other substances, for example, tissue extracts, cell culture supernatants from humans or mammals (e.g., mice, rats, swine, bovine, sheep, monkeys, etc.) or low molecular synthetic compound. For example, the tissue extract, or cell culture supernatant, is added to the receptor protein of the present invention while assaying the cell-stimulating activities and fractionating to finally obtain a single ligand.

Specifically, the agonist determination method of the present invention is a method of determining a compound or its salt having the cell stimulating activity mediated by the GPCR of the invention, which involves constructing the expression system of recombinant GPCR of the invention and assaying the cell stimulating activity mediated by the GPCR of the invention, by using the receptor-binding assay system using the aforesaid expression system.

More specifically, the present invention provides the following determination methods:

(1) a method of determining the agonist to the GPCR of the invention, which comprises assaying the intracellular cAMP production suppressing activity in the case where a test compound is brought into contact with cells containing the GPCR of the invention; and

(2) a method of determining the agonist to the GPCR of the invention, which comprises assaying the intracellular cAMP production suppressing activity mediated by the GPCR of the invention in the case where a test compound is brought into contact with a receptor protein expressed on the cell membrane by culturing a transformant containing a DNA encoding the GPCR of the invention.

In particular, it is preferred to carry out the test described above after confirmation that the test compound binds to the GPCR of the invention.

Where the cell containing the GPCR of the invention is used in the agonist determination methods of the present invention, the cell may be fixed using glutaraldehyde, formalin, etc. The fixation can be made by publicly known methods.

The membrane fraction of the cell containing the GPCR of the invention means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the GPCR of the invention expressed and membrane components such as cell-derived phospholipids, membrane proteins, etc.

The amount of the GPCR of the invention in the cell or the cell membrane fraction is preferably $10^3$ to $10^8$ molecules per cell, more preferably $10^5$ to $10^7$ molecules per cell. As the level of expression increases, the ligand binding activity per unit of membrane fraction (specific activity) increases so that not only the highly sensitive screening system can be constructed but also large quantities of samples can be assayed with the same lot.

To perform the agonist determination method of the present invention, the intracellular cAMP production suppressing activity mediated by the GPCR of the invention can be assayed by publicly known methods or using assay kits commercially available. Specifically, the cells containing the GPCR of the invention are first cultured on a multiwell plate, etc. Prior to the agonist determination, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the indicator substance (e.g., cAMP, etc.) for the cell-stimulating activities due to a degrading enzyme contained in the cells, an inhibitor against such a degrading enzyme may be added prior to the assay. For detecting activities such as the cAMP production suppression, etc., the baseline production in the cells is increased by forskolin or the like and the suppressing effect on the increased baseline production may then be detected.

The kit for agonist determination of the present invention contains the cell containing the GPCR of the invention or a membrane fraction of the cell.

The agonist to the GPCR of the invention thus determined binds to the GPCR of the invention to regulate its physiological functions and thus can be used as a central or peripheral neural function-regulating agent or as a preventive/therapeutic agent for diseases associated with the functions of the GPCR of the invention. Specifically, the agonist is useful as an antianxiety drug, a hypnotic sedative, a muscle relaxant, an anesthetic fortifier, or as a preventive/therapeutic drug for diseases such as convulsion, dependence, schizophrenia, epilepsy, psychosomatic disorders, incontinence, neurogenic hypertension, threatened abortion, threatened premature delivery, male infertility, autonomic imbalance, etc. Furthermore, the agonist to the GPCR of the invention can be used as a preventive/therapeutic agent for cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia, palsy, etc.

(6) Method of screening a compound (agonist, antagonist, etc.) that changes the binding properties of the GPCR of the invention to its ligand and pharmaceuticals comprising the compound that changes the binding properties of the GPCR of the invention to its ligand

By using the GPCR of the invention, or by constructing a recombinant of the GPCR of the invention expression system and using the receptor-binding assay system via the expression system, the compound (e.g., peptide, protein, a non-peptide compound, a synthetic compound, a fermentation product, etc.) or salts thereof that change the binding properties of β-alanine or L-carnosine as a ligand to the GPCR of the invention can be screened efficiently.

Examples of these compounds include (a) a compound showing the cell stimulating activities mediated by the GPCR of the invention (a so-called agonist to the GPCR of the invention), (b) a compound having no such cell stimulating activities (a so-called antagonist to the GPCR of the invention), (c) a compound that potentiates the binding affinity of β-alanine or L-carnosine to the GPCR of the invention, or (d) a compound that decreases the binding affinity of β-alanine or L-carnosine to the GPCR of the invention, and the like.

That is, the present invention provides a method of screening a compound or its salt that changes the binding properties of the GPCR of the invention to β-alanine or L-carnosine, which comprises comparing the following two cases: (i) the case wherein the GPCR of the invention is brought in contact with β-alanine or L-carnosine; and (ii) the case wherein the GPCR of the invention is brought in contact with β-alanine or L-carnosine and a test compound.

According to the screening method of the present invention, the method is characterized by assaying, e.g., the binding amount of β-alanine or L-carnosine to the GPCR of the invention, the cell-stimulating activities, etc. in (i) and (ii) and comparing (i) and (ii).

Examples of the cell-stimulating activities include the activities that promote or suppress arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc. Among them, the intracellular cAMP production suppressing activity and the like are preferred.

More specifically, the present invention provides the following methods.

a) A method of screening a compound or a salt thereof that changes the binding properties of β-alanine or L-carnosine to the GPCR, which comprises measuring the binding amount of labeled β-alanine or L-carnosine to the GPCR of the invention in the case wherein the labeled β-alanine or L-carnosine is brought in contact with the GPCR of the invention and in the case wherein the labeled β-alanine or L-carnosine and a test compound are brought in contact with the GPCR of the invention, and comparing the cases.

b) A method of screening a compound or a salt thereof that changes the binding properties of β-alanine or L-carnosine to the GPCR of the invention, which comprises measuring the binding amount of labeled β-alanine or L-carnosine to a cell containing the GPCR of the invention or a membrane fraction of the cell, in the case wherein the labeled β-alanine or L-carnosine is brought in contact with the cell containing the GPCR of the invention or the membrane fraction and in the case wherein the labeled β-alanine or L-carnosine and a test compound are brought in contact with the cell containing the GPCR of the invention or its membrane fraction, and comparing the cases.

c) A method of screening a compound or a salt thereof that changes the binding properties of β-alanine or L-carnosine to the GPCR, which comprises measuring the amount of labeled β-alanine or L-carnosine bound to the GPCR of the invention, in the case wherein the labeled β-alanine or L-carnosine is brought in contact with the GPCR of the invention expressed on a cell membrane by culturing a transformant containing the DNA of the present invention and in the case wherein the labeled β-alanine or L-carnosine and a test compound are brought in contact with the GPCR of the invention expressed on the cell membrane by culturing a transformant containing the DNA of the present invention, and comparing the cases.

d) A method of screening a compound or its salt that changes the binding properties of the ligand to the GPCR, which comprises assaying the cell stimulating activities mediated by the GPCR of the invention in the case wherein a compound (e.g., β-alanine or L-carnosine, etc.) that activates the GPCR of the invention is brought in contact with a cell containing the GPCR of the invention and in the case wherein said compound that activates the GPCR of the invention and a test compound are brought in contact with the cell containing the GPCR of the invention, and comparing the cell stimulating activities between the two cases.

e) A method of screening a compound or a salt thereof that changes the binding properties of β-alanine or L-carnosine to the GPCR of the invention, which comprises assaying the receptor protein-mediated cell stimulating activities in the case wherein a compound (e.g., β-alanine or L-carnosine, etc.) that activates the GPCR of the invention is brought in contact with the GPCR of the invention expressed on a cell membrane by culturing a transformant containing the DNA of the present invention and in the case wherein said compound that activates the GPCR of the invention and a test compound are brought in contact with the GPCR of the invention expressed on a cell membrane by culturing a transformant containing the DNA of the present invention, and comparing the cell stimulating activities between the two cases.

Further as the ligand, a compound or its salt that changes the binding properties of β-alanine or L-carnosine to the GPCR of the invention can also be used, in place of β-alanine or L-carnosine. The compound or its salt that changes the binding properties of β-alanine or L-carnosine to the GPCR of the invention can be obtained by carrying out the screening methods later described, using as the ligand, e.g., β-alanine or L-carnosine. In the following screening methods, the ligand is briefly referred to as β-alanine or L-carnosine, including the compound or its salt that changes the binding properties of β-alanine or L-carnosine to the GPCR of the invention.

Hereinafter the screening method of the present invention will be described more specifically.

First, the GPCR of the invention, which is used for the screening method of the present invention, may be any one so long as it contains the GPCR of the invention described above, though membrane fractions from mammalian organs are preferably employed. Since it is very difficult to obtain human-derived organs especially, human-derived TGR7, etc. expressed abundantly by use of recombinants are suitable for use in the screening.

In manufacturing the GPCR of the invention, the methods described above can be used, and the DNA of the present invention is preferably expressed on mammalian cells or insect cells. As the DNA fragment encoding the target protein region, a complementary DNA may be used but is not limited thereto. For example, gene fragments or a synthetic DNA may also be used. In order to introduce the DNA fragment encoding the GPCR of the invention into host animal cells and express the same efficiently, the DNA fragment is preferably incorporated into a polyhedron promoter of nuclear polyhedrosis virus (NPV) belonging to the Baculovirus, an SV40-derived promoter, a promoter of retrovirus, a metallothionein promoter, a human heat shock promoter, a cytomegalovirus promoter, SRα promoter, etc. at the downstream thereof. The quantity and quality of the thus expressed receptors can be examined by a publicly known method, for example, by the method described in the literature [Nambi, P. et al., J. Biol. Chem., 267, 19555-19559, 1992].

Accordingly, in the screening method of the present invention, the substance containing the GPCR of the invention may be the GPCR of the invention purified by publicly known methods, or a cell containing the said the GPCR of the invention or a membrane fraction of the cell containing the GPCR may be used as well.

Where the cell containing the GPCR of the invention is used in the screening method of the present invention, the cell may be fixed with glutaraldehyde, formalin, etc. The fixation may be carried out by a publicly known method.

The cell containing the GPCR of the invention refers to a host cell expressing the GPCR. Examples of such a host cell include Escherichia coli, Bacillus subtilis, yeast, insect cells, animal cells, etc.

The membrane fraction of the cell containing the GPCR of the invention means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the GPCR of the invention expressed and membrane components such as cell-derived phospholipids, membrane proteins, etc.

The amount of the GPCR in a cell containing the GPCR of the invention or the cell membrane fraction is preferably $10^3$ to $10^8$ molecules per cell, more preferably $10^5$ to $10^7$ molecules per cell. As the level of expression increases, the ligand binding activity per unit of membrane fraction (specific activity) increases so that not only the highly sensitive screening system can be constructed but also large quantities of samples can be assayed with the same lot.

To perform a) through c) above for screening the compound that changes the binding properties of β-alanine or L-carnosine to the GPCR of the invention, an appropriate fraction of the GPCR of the invention and labeled β-alanine or L-carnosine are required.

The fraction of the GPCR of the invention is preferably a fraction of the GPCR of the invention of naturally occurring type or fraction of the GPCR of the invention of recombinant type having an activity equivalent thereto. Herein, the equivalent activity is intended to mean the ligand binding activity or the signal transduction activity, which is equivalent to the activity possessed by the GPCR of the invention of naturally occurring type.

As labeled β-alanine or L-carnosine, labeled β-alanine or L-carnosine, a labeled β-alanine analogue or labeled L-carnosine and the like are employed. For example, there are used β-alanine or L-carnosine labeled with [$^3$H], [$^{125}$I], [$^{14}$C] [$^{35}$S], etc.

Specifically, the compound that changes the binding properties of β-alanine or L-carnosine to the GPCR of the invention is screened by the following procedures. First, a preparation of the GPCR of the invention is prepared by suspending a cell containing the GPCR of the invention or a membrane fraction of the cell in a buffer appropriate for use in the screening method. Any buffer can be used so long as it does not interfere the binding affinity of β-alanine or L-carnosine to the GPCR of the invention, including a phosphate buffer or a Tris-HCl buffer, having pH of 4 to 10 (preferably pH of 6 to 8), etc. For the purpose of minimizing non-specific binding, a surfactant such as CHAPS, Tween-80™ (Kao-Atlas Inc.), digitonin, deoxycholate, etc., may optionally be added to the buffer. Further for the purpose of suppressing the degradation of the receptor protein or ligand by a protease, a protease inhibitor such as PMSF, leupeptin, E-64 (manufactured by Peptide Institute, Inc.), pepstatin, etc. may also be added. A given amount (5,000 cpm to 500,000 cpm) of labeled β-alanine or L-carnosine is added to 0.01 ml to 10 ml of the receptor protein solution, in which $10^{-4}$ M to $10^{-10}$ M of a test compound is co-present. To determine the amount of non-specific binding (NSB), a reaction tube containing unlabeled β-alanine or L-caenosine in a large excess is also provided. The reaction is carried out at approximately 0°C to 50°C, preferably approximately 4°C to 37°C for about 20 minutes to about 24 hours, preferably about 30 minutes to 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. When nonspecific binding (NSB) is subtracted from the count ($B_0$) where any antagonizing substance is absent and the resulting count ($B_0$ minus NSB) is made 100%, the test compound showing the specific binding amount (B minus NSB) of, e.g., 50% or less may be selected as a candidate compound.

The method d) or e) described above for screening the compound that changes the binding properties of β-alanine or L-carnosine to the GPCR of the invention can be performed as follows. For example, the cell-stimulating activities mediated by the GPCR of the invention (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc., especially the intracellular cAMP production suppressing activity) can be determined by a publicly known method, or using an assay kit commercially available.

Specifically, the cells containing the GPCR of the invention are first cultured in a multiwell plate, etc. Prior to screening, the medium is replaced with fresh medium or with an appropriate non-cytotoxic buffer, followed by incubation for a given period of time in the presence of a test compound, etc. Subsequently, the cells are extracted or the supernatant is recovered and the resulting product is quantified by appropriate procedures. Where it is difficult to detect the production of the cell-stimulating activity indicator (e.g., arachidonic acid, etc.) due to a degrading enzyme contained in the cells, an inhibitor against such as a degrading enzyme may be added prior to the assay. For detecting the activities such as the cAMP production suppression activity, the baseline production in the cells is increased by forskolin or the like and the suppressing effect on the increased baseline production can be detected.

For screening through the assay for the cell stimulating activities, cells, in which an appropriate type of the GPCR of the invention has been expressed, are necessary. Preferred cells, in which the GPCR of the invention has been expressed, are a cell line containing the GPCR of the invention of naturally occurring type and the aforesaid cell line, in which the GPCR of the invention of recombinant type has been expressed.

Examples of the test compounds include peptides, proteins, non-peptide compounds, synthetic compounds, fermentation products, cell extracts, plant extracts, animal tissue extracts, etc. These test compounds may be either novel or publicly known compounds.

The test compound which is preferably used is a compound designed to bind to the ligand-binding pocket, based on the atomic coordinate and the position of the ligand-binding pocket in the active site of the GPCR of the invention. The atomic coordinate and the position of the ligand-binding pocket in the active site of the GPCR of the invention can be determined by publicly known methods or modifications thereof.

Following the method of screening the agonist to the GPCR of the invention described above, it can be confirmed whether the compound that changes the binding properties of β-alanine or L-carnosine to the GPCR of the invention is either an agonist or an antagonist.

The kit for screening the compound or its salt that changes the binding properties of β-alanine or L-carnosine to the GPCR of the invention is a kit comprising the GPCR of the invention, a cell containing the GPCR of the invention, or a membrane fraction of the cell containing the GPCR of the invention, and the like.

Examples of the screening kit of the present invention include the following.

1. Reagent for screening

a) Assay buffer and wash buffer

Hanks' balanced salt solution (manufactured by Gibco, Inc.) supplemented with 0.05% bovine serum albumin (manufactured by Sigma, Inc.)

The solution is sterilized by filtration through a 0.45 μm filter, and stored at 4°C or may be prepared at use.

b) Preparation of the GPCR of the invention

CHO cells wherein the GPCR of the invention has been expressed are passaged in a 12-well plate at a density of $5 \times 10^5$ cells/well followed by culturing at 37°C under 5% $CO_2$ and 95% air for 2 days.

c) Labeled β-alanine or L-carnosine

An aqueous solution of β-alanine or L-carnosine labeled with commercially available [$^3$H], [$^{125}$I], [$^{14}$C], [$^{35}$S], etc. is stored at 4°C or -20°C, and diluted to 1 μM with the assay buffer upon use.

d) Standard solution of β-alanine or L-carnosine

β-Alanine or L-carnosine is dissolved in and adjusted to 1 mM with PBS containing 0.1% bovine serum albumin (manufactured by Sigma, Inc.) and stored at -20°C.

2. Assay method

a) CHO cells wherein the GPCR of the invention has been expressed are cultured in a 12-well culture plate and washed twice with 1 ml of the assay buffer, and 490 μl of the assay buffer is added to each well.

b) After adding 5 μl of $10^{-3}$ - $10^{-10}$ M test compound solution, 5 μl of labeled β-alanine or L-carnosine is added to the mixture, and the cells are incubated at room temperature for an hour. To determine the amount of the non-specific binding, 5 μl of β-alanine or L-carnosine is added in place of the test compound.

c) The reaction solution is removed, and the wells are washed 3 times with the washing buffer. The labeled ligand bound to the cells is dissolved in 0.2N NaOH-1% SDS, and mixed with 4 ml of liquid scintillator A (manufactured by Wako Pure Chemical Industries, Ltd.)

d) The radioactivity is measured using a liquid scintillation counter (manufactured by Beckman Co.), and the percent maximum binding (PMB) is calculated by the equation below.

$$PMB = [(B - NSB)/(B_0 - NSB)] \times 100$$

PMB :     Percent maximum binding
B :     Value obtained in the presence of a test compound
NSB :     Non-specific binding
$B_0$ :     Maximum binding

The compound or its salt, which is obtained by using the screening methods or the screening kits of the present invention, is the compound that changes the binding properties of β-alanine or L-carnosine to the GPCR of the invention. Specifically, the compound is: (a) a compound having the cell-stimulating activities mediated by the G protein-coupled receptor (a so-called agonist to the GPCR of the invention); (b) a compound having no cell stimulating activity (a so-called antagonist to the GPCR of the invention); (c) a compound that potentiates the binding affinity of β-alanine or L-carnosine to the GPCR of the invention; or (d) a compound that reduces the binding affinity of β-alanine or L-carnosine to the GPCR of the invention.

These compounds may be peptides, proteins, non-peptide compounds, synthetic compounds, fermentation products, and may be novel or known compounds.

The compound or its salt obtained by using the screening method or the screening kit of the present invention is useful as a central or peripheral neural function-regulating agent.

Since the agonists to the GPCR of the invention have the same physiological activities as β-alanine or L-carnosine, which is a ligand to the GPCR of the invention, the agonists are useful as safe and low toxic pharmaceuticals, correspondingly to the physiological activities possessed by β-alanine or L-carnosine.

Since the antagonists to the GPCR of the invention can suppress the physiological activities possessed by β-alanine or L-carnosine, which is a ligand to the GPCR of the invention, the antagonists are useful as safe and low toxic pharmaceuticals to suppress the physiological activities of β-alanine or L-carnosine.

The compound that potentiates the binding affinity of β-alanine or L-camosine to the GPCR of the invention can potentiate the physiological activities possessed by β-alanine or L-carnosine as the ligand to the GPCR of the invention. Thus, the compound is useful as a safe and low toxic pharmaceutical correspondingly to the physiological activities β-alanine or L-carnosine possesses.

The compound that reduces the binding affinity of β-alanine or L-carnosine to the GPCR of the invention can reduce the physiological activities possessed by β-alanine or L-carnosine as the ligand to the GPCR of the invention. Thus, the compound is useful as a safe and low toxic pharmaceutical to suppress the physiological activities β-alanine or L-carnosine possesses.

Specifically, (i) the agonist to the GPCR of the invention or (ii) the compound or its salt that potentiates the binding affinity of β-alanine or L-carnosine to the GPCR of the invention, which is obtained by using the screening methods or screening kits of the present invention, is useful as an antianxiety drug, a hypnotic sedative, a muscle relaxant or an anesthetic fortifier, or as a preventive/therapeutic drug for diseases such as convulsion, dependence, schizophrenia, epilepsy, psychosomatic disorders, incontinence, neurogenic hypertension, threatened abortion, threatened premature delivery, male infertility, autonomic imbalance, etc. In addition, (i) the agonist to the GPCR of the invention or (ii) the compound or its salt that potentiates the binding affinity of β-alanine or L-carnosine to the GPCR of the invention, which is obtained by using the screening methods or screening kits of the present invention, is useful as a preventive/therapeutic drug for diseases such as cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia, palsy, etc.

(i) The antagonist to the GPCR of the invention or (ii) the compound or its salt that reduces the binding affinity of β-alanine or L-carnosine to the GPCR of the invention, which is obtained by using the screening methods or screening kits of the present invention, is useful as a preventive/therapeutic agent for, e.g., ataxia, paralysis, depression, delayed awakening, deficits in memory and learning, hypotension, dysuria, hypertrophy of bladder, dyspnea, dependence or sensory paralysis, etc.

Where the compound or its salt, which is obtained by using the screening methods or screening kits of the present invention, is used as the pharmaceutical composition described above, the compound or its salt can be prepared into a pharmaceutical preparation in a conventional manner.

For example, the compound can be used orally in the form of tablets which may be tablets, if necessary, coated with sugar, capsules, elixirs, microcapsules, etc., or parenterally in the form of injectable preparations such as a sterile solution or a suspension in water or with other pharmaceutically acceptable liquid. These preparations can be manufactured, e.g., by mixing the compound, with a physiologically acceptable known carrier, flavoring agent, excipient, vehicle, antiseptic, stabilizer, binder, etc., in a unit dosage form required in a generally accepted manner applied to making pharmaceutical preparations. The active ingredient in the preparation is controlled in such an amount that an appropriate dose is obtained within the specified range given.

Additives miscible with tablets, capsules, etc. include a binder such as gelatin, corn starch, tragacanth or gum arabic, an excipient such as crystalline cellulose, a swelling agent such as corn starch, gelatin, alginic acid, etc., a lubricant such as magnesium stearate, a sweetening agent such as sucrose, lactose or saccharin, a flavoring agent such as peppermint, akamono oil or cherry, and the like. When the unit dosage is in the form of capsules, liquid carriers such as oils and fats may further be used together with the additives described above. A sterile composition for injection may be formulated following a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline, an isotonic solution containing glucose and other auxiliary agents (e.g., D-sorbitol, D-mannitol, sodium chloride, etc.) or the like, which may be used in combination with an appropriate dissolution aid such as an alcohol (e.g., ethanol), a polyalcohol (e.g., propylene glycol, polyethylene glycol), a nonionic surfactant (e.g., polysorbate 80™ and HCO-50), etc. As an oily medium, for example, sesame oil, soybean oil or the like may be used, and may be used in combination with a dissolution aid such as benzyl benzoate, benzyl alcohol, etc.

Furthermore, the drugs described above may also be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the agonist to the GPCR of the invention may vary depending on subject to be administered, target organ, conditions, route for administration, etc.; in oral administration, the dose for the patient with schizophrenia (as 60 kg body weight) is normally about 0.1 mg to about 100 mg, preferably about 1.0 to about 50 mg, and more preferably about 1.0 to about 20 mg per day. In parenteral administration, the single dose may vary depending on subject to be administered, target organ, conditions, method for administration, etc. but it is advantageous to administer the active ingredient intravenously to the patient with schizophrenia (as 60 kg) in a daily dose of about 0.01 to about 30 mg, preferably about 0.1 to about 20 mg, and more preferably about 0.1 to about 10 mg. For other animal species, the corresponding dose as converted per 60 kg body weight can be administered.

(6) Methods for elucidation of the action mechanism of various drugs

By using the GPCR of the invention, it can be confirmed whether or not various drugs exhibit their pharmacological effects mediated by the GPCR of the invention.

That is, the present invention provides the following methods.

(1) A method of confirmation that a central or peripheral neural function-regulating agent or a preventive/therapeutic agent for anxiety, insomnia, excitement, muscular atrophy, anesthesia sensitivity, convulsion, dependence, schizophrenia, epilepsy, psychosomatic disorders, incontinence, neurogenic hypertension, threatened abortion, threatened premature delivery, male infertility, autonomic imbalance, cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia, palsy, ataxia, paralysis, depression, delayed awakening, deficits in memory and learning, hypotension, dysuria, hypertrophy of bladder, dyspnea or sensory paralysis binds to the receptor protein or its salt, which comprises using the GPCR of the invention.

(2) A method of confirmation that a central or peripheral neural function-regulating agent or a preventive/therapeutic agent for anxiety, insomnia, excitement, muscular atrophy, anesthesia sensitivity, convulsion, dependence, schizophrenia, epilepsy, psychosomatic disorders, incontinence, neurogenic hypertension, threatened abortion, threatened premature delivery, male infertility, autonomic imbalance, cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy is the agonist to the receptor protein or its salt, which comprises using the GPCR of the invention.

(3) A method of confirmation that a central or peripheral neural function-regulating agent or a preventive/therapeutic agent for ataxia, paralysis, depression, delayed awakening, deficits in memory and learning, hypotension, dysuria, hypertrophy of bladder, dyspnea, dependence or sensory paralysis is the antagonist to the receptor protein or its salt, which comprises using the GPCR of the invention.

(4) The screening method according to (1) to (3), wherein the binding amount of each drug to the GPCR of the invention is measured when the drug is brought in contact with the GPCR of the invention.

This confirmation method can be performed by using the drug described above in lieu of the test compound in the aforesaid method of screening the compound that changes the binding properties of the ligand to the GPCR of the invention.

The kit used for the confirmation method of the present invention comprises the drug described above in place of the test compound, in the aforesaid kit for screening the compound that changes the binding properties of the ligand to the GPCR of the invention.

By using the confirmation method of the present invention as such, it can be confirmed that various drugs commercially available or under development exhibit their pharmacological effects mediated by the GPCR of the invention.

(7) Pharmaceutical comprising the compound that changes the amount of the GPCR of the invention or its partial peptide in cell membrane

The antibody of the present invention is capable of specifically recognizing the GPCR of the invention and can be used for screening the compound that changes the amount of the GPCR of the invention in the cell membrane.
That is, the present invention provides the following methods:

(i) a method of screening the compound that changes the amount of the GPCR of the invention in the cell membrane, which comprises measuring the amount of the GPCR of the invention contained in a) blood, b) particular organs or c) a cell membrane fraction isolated after disrupting tissues or cells isolated from the organs of non-human mammals;
(ii) a method of screening the compound that changes the amount of the GPCR of the invention in the cell membrane, which comprises disrupting transformants, etc. expressing the GPCR of the invention, isolating the cell membrane fraction and quantifying the GPCR of the invention contained in the cell membrane fraction; and,
(iii) a method of screening the compound that changes the amount of the GPCR of the invention in the cell membrane, which comprises preparing a slice of a) blood, b) particular organs or c) tissues, cells, etc. isolated from organs of non-human mammals and quantifying the stained receptor protein on the cell surface using immunostaining assay thereby to confirm the protein on the cell membrane.

The present invention provides:

(iv) a method of screening the compound that changes the amount of the GPCR of the invention in the cell membrane, which comprises preparing a slice of a transformant expressing the GPCR of the invention and quantifying the stained receptor protein on the cell surface using immunostaining assay thereby to confirm the protein on the cell membrane.

Specifically, the GPCR of the invention contained in the cell membrane fraction can be measured as follows.
(i) Normal or disease models of non-human mammals (e.g., mice, rats, rabbits, sheep, swine, bovine, cats, dogs, monkeys, more specifically, rats with dementia, obese mice, rabbits with arteriosclerosis, tumor-bearing mice, etc.) receive administration of a drug (e.g., an anti-dementia drug, a hypotensive drug, an anticancer agent, an antiobestic drug, etc.) or physical stress (e.g., soaking stress, electric shock, light and darkness, low temperature, etc.), and the blood, particular organs (e.g., brain, liver, kidney, etc.), or tissues or cells isolated from the organs are obtained after a specified period of time. The obtained organs, tissues, cells or the like are suspended in, for example, an appropriate buffer (e.g., Tris hydrochloride buffer, phosphate buffer, HEPES buffer, etc.), and the organs, tissues or cells are disrupted, and the cell membrane fraction is obtained using surfactants (e.g., Triton-X 100™, Tween 20™) and further using techniques such as centrifugal separation, filtration, column fractionation, etc.
The membrane fraction of the cell containing the GPCR of the invention means a fraction abundant in cell membrane obtained by cell disruption and subsequent fractionation by publicly known methods. Cell disruption methods include cell squashing using a Potter-Elvehjem homogenizer, disruption using a Waring blender or Polytron (manufactured by Kinematica Inc.), disruption by ultrasonication, disruption by cell spraying through thin nozzles under an increased pressure using a French press, or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the GPCR of the invention expressed and membrane components such as cell-derived phospholipids, membrane proteins, etc.
The GPCR of the invention contained in the cell membrane fraction can be quantified by, for example, the sandwich immunoassay, western blot analysis, etc. using the antibody of the present invention.
The sandwich immunoassay can be performed as described above, and the western blot can be performed by publicly known methods.
(ii) Transformants expressing the GPCR of the invention are prepared by the method described above, and the GPCR of the invention contained in the cell membrane fraction can be quantified.
The compound that changes the amount of the GPCR of the invention in cell membranes can be screened as follows.
(i) Normal non-human mammals or disease models of non-human mammals are administered with a test compound at a specified period of time before (30 minutes to 24 hours before, preferably 30 minutes to 12 hours before, more preferably 1 hour to 6 hours before), at a specified time after (30 minutes to 3 days after, preferably 1 hour to 2 days after, more preferably 1 hour to 24 hours after), or simultaneously with a drug or physical stress. At a specified time (30 minute to 3 days, preferably 1 hour to 2 days, more preferably 1 hour to 24 hours) after administration of the test compound, the amount of the GPCR of the invention in the cell membranes can be quantified.

(ii) Transformants are cultured in a conventional manner and a test compound is mixed in the culture medium. After a specified time (after 1 day to 7 days, preferably after 1 day to 3 days, more preferably after 2 to 3 days), the amount of the GPCR of the invention in the cell membranes can be quantified.

Specifically, the GPCR of the invention contained in cell membrane fractions is confirmed as follows.

(iii) Normal non-human mammals or disease models of non-human mammals (e.g., mice, rats, rabbits, sheep, swine, bovine, cats, dogs, monkeys, etc., more specifically, rat with dementia, obese mouse, rabbit with arteriosclerosis, tumor-bearing mouse, etc.) are administered with drugs (e.g., anti-dementia agents, hypotensive agents, anticancer agents, antiobestic agents, etc.) or physical stress (e.g., soaking stress, electric shock, light and darkness, low temperature, etc.) or the like, and blood or particular organ (e.g., brain, liver, kidney, etc.), or the tissues or cells isolated from the organ are obtained after a specified period of time. Tissue sections are prepared from the thus obtained organs, tissues, cells, etc. in a conventional manner followed by immunostaining with the antibody of the present invention. The staining intensity of the receptor protein on the cell surface is quantified to confirm the protein on the cell membrane, whereby the amount of the GPCR of the invention on the cell membrane can be confirmed quantitatively or qualitatively.

(iv) The confirmation can also be made by the similar method, using transformants expressing the GPCR of the invention.

The compounds or its salts obtained by the screening methods of the present invention are the compounds that have the action of changing the amount of the GPCR of the invention in the cell membranes. Specifically, these compounds are: (a) compounds that increase the amount of the GPCR of the invention in the cell membranes thereby to potentiate the cell stimulating activities mediated by the GPCR of the invention and (b) compounds that decrease the amount of the GPCR of the invention in the cell membranes thereby to attenuate the cell stimulating activities.

The compounds may be peptides, proteins, non-peptide compounds, synthetic compounds, fermentation products, and may be novel or publicly known compounds.

The compound obtained using the screening method of the present invention is useful as a central or peripheral neural function-regulating drug.

The compounds that increase the amount of the GPCR of the invention in the cell membranes thereby to enhance the cell-stimulating activities are useful as safe and low toxic preventive/therapeutic drugs for diseases associated with dysfunction of the GPCR of the invention.

The compounds that decrease the amount of the GPCR of the invention in the cell membranes thereby to attenuate the cell-stimulating activities are useful as safe and low toxic preventive/therapeutic drugs for diseases caused by overexpression of the GPCR of the invention.

Specifically, the compound or its salt that increases the amount of the GPCR of the invention can be used as an antianxiety drug, a hypnotic sedative, a muscle relaxant, an anesthetic fortifier, or as a preventive/therapeutic agent for diseases such as convulsion, dependence, schizophrenia, epilepsy, psychosomatic disorders, incontinence, neurogenic hypertension, threatened abortion, threatened premature delivery, male infertility, autonomic imbalance, etc. Furthermore, the compound or its salt that increases the amount of the GPCR of the invention can be used as a preventive/therapeutic agent for diseases such as cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia, palsy, etc.

The compound or its salt that decreases the amount of the GPCR of the invention can be used as a preventive/ therapeutic agent for diseases, e.g., ataxia, paralysis, depression, delayed awakening, deficits in memory and learning, hypotension, dysuria, hypertrophy of bladder, dyspnea, dependence or sensory paralysis, etc.

Where the compound or its salt obtained by using the screening methods of the present invention is used as a pharmaceutical composition, the compound or its salt can be prepared into a pharmaceutical preparation in a conventional manner.

For example, the compound can be used orally in the form of tablets which may be tablets, if necessary, coated with sugar, capsules, elixirs, microcapsules, etc., or parenterally in the form of injectable preparations such as a sterile solution or a suspension in water or with other pharmaceutically acceptable liquid. These preparations can be manufactured, e.g., by mixing the compound, with a physiologically acceptable known carrier, flavoring agent, excipient, vehicle, antiseptic, stabilizer, binder, etc., in a unit dosage form required in a generally accepted manner applied to making pharmaceutical preparations. The active ingredient in the preparation is controlled in such an amount that an appropriate dose is obtained within the specified range given.

Additives miscible with tablets, capsules, etc. include a binder such as gelatin, corn starch, tragacanth or gum arabic, an excipient such as crystalline cellulose, a swelling agent such as corn starch, gelatin, alginic acid, etc., a lubricant such as magnesium stearate, a sweetening agent such as sucrose, lactose or saccharin, a flavoring agent such as peppermint, akamono oil or cherry, and the like. When the unit dosage is in the form of capsules, liquid carriers such as oils and fats may further be used together with the additives described above. A sterile composition for injection may be formulated following a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition. Examples of an aqueous medium for injection include physiological saline, an isotonic solution containing glucose and other auxiliary agents (e.g., D-sorbitol, D-mannitol, sodium chloride, etc.) or the like, which may be used in combination with an appropriate dissolution aid such as an alcohol (e.g., ethanol), a polyalcohol (e.g., propylene glycol, polyethylene glycol), a nonionic surfactant (e. g., polysorbate 80™ and HCO-50), etc. As an oily medium, for example, sesame oil, soybean oil or the like may be used, which can be used in combination with a dissolution aid such as benzyl benzoate, benzyl alcohol, etc.

Furthermore, the preventive/therapeutic drug described above may also be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

Since the thus obtained pharmaceutical preparation is safe and low toxic, the preparation can be administered to human or mammals (e.g., rats, mice, rabbits, sheep, swine, bovine, cats, dogs, monkeys, etc.).

The dose of the compound or its salt that increases the amount of the GPCR of the invention in the cell membranes may vary depending on subject to be administered, target organ, conditions, methods for administration, etc.; in oral administration, the dose for the patient with schizophrenia (as 60 kg body weight) is normally about 0.1 mg to about 100 mg, preferably about 1.0 to about 50 mg, and more preferably about 1.0 to about 20 mg per day. In parenteral administration, the single dose may vary depending on subject to be administered, target organ, conditions, methods for administration, etc. but it is advantageous to administer the active ingredient intravenously to the patient with schizophrenia (as 60 kg body weight) in a daily dose of about 0.01 to about 30 mg, preferably about 0.1 to about 20 mg, and more preferably about 0.1 to about 10 mg. For other animal species, the corresponding dose as converted per 60 kg body weight can be administered.


(8) Pharmaceutical comprising the antibody to the GPCR of the invention


The neutralizing activity of the antibody to the GPCR of the invention means the activity of inactivating the signal transduction function in which the GPCR takes part. Thus, when the antibody has the neutralizing activity, the antibody can inactivate signal transduction in which the GPCR of the invention takes part, for example, the cell stimulating activities mediated by the GPCR of the invention (e.g., the activities that promote or suppress arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.).

Therefore, the neutralizing antibody to the GPCR of the invention can be used as a central or peripheral neural function-regulating agent or as a preventive/therapeutic agent for diseases caused by overexpression of the GPCR (for example, diseases such as ataxia, paralysis, depression, delayed awakening, deficits in memory and learning, hypotension, dysuria, hypertrophy of bladder, dyspnea, dependence or sensory paralysis, etc.)


(9) Pharmaceutical comprising the antisense DNA of the present invention


The antisense DNA of the present invention can be used as a central or peripheral neural function-regulating agent or as a preventive/therapeutic agent for diseases caused by overexpression of the GPCR of the invention (for example, diseases such as ataxia, paralysis, depression, delayed awakening, deficits in memory and learning, hypotension, dysuria, hypertrophy of bladder, dyspnea, dependence or sensory paralysis, etc.).

For example, where the antisense DNA is used, the antisense DNA itself is administered; alternatively, the antisense DNA is inserted into an appropriate vector such as retrovirus vector, adenovirus vector, adenovirus-associated virus vector, etc. and then administered in a conventional manner. The antisense DNA may also be administered as naked, or with adjuvants to assist its uptake by gene gun or through a catheter such as a catheter with a hydrogel.

In addition, the antisense DNA may also be used as an oligonucleotide probe for diagnosis to investigate the presence of the DNA of the present invention or the state of its expression in tissues or cells.


(10) Preparation of animal bearing the DNA of the present invention


The present invention provides a non-human mammal bearing DNA which is exogenous (hereinafter briefly referred to as the exogenous DNA of the present invention) or its variant DNA (sometimes briefly referred to as the exogenous variant DNA of the present invention).

That is, the present invention provides:

[1] A non-human mammal bearing the exogenous DNA of the present invention or its variant DNA;
[2] The mammal according to [1], wherein the non-human mammal is a rodent;
[3] The mammal according to [2], wherein the rodent is mouse or rat; and,
[4] A recombinant vector containing the exogenous DNA of the present invention or its variant DNA and capable of expressing in a mammal; etc.

The non-human mammal bearing the exogenous DNA of the present invention or its variant DNA (hereinafter briefly referred to as the DNA transgenic animal of the present invention) can be produced by transferring a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method, etc. Also, it is possible to transfer the exogenous DNA of the present invention into a somatic cell, a living organ, a tissue cell, or the like by the DNA transfer, and utilize the transformant for cell culture, tissue culture, etc. In addition, these cells may be fused with the above-described germinal cell by a publicly known cell fusion method to prepare the DNA transgenic animal of the present invention.

Examples of the non-human mammal that can be used include bovine, swine, sheep, goat, rabbits, dogs, cats, guinea pigs, hamsters, mice, rats, etc. Above all, preferred are rodents, especially mice (e.g., C57Bl/6 strain, DBA2 strain, etc. for a pure line and for a cross line, $B6C3F_1$ strain, $BDF_1$ strain $B6D2F_1$ strain, BALB/c strain, ICR strain, etc.), rats (Wistar, SD, etc.) or the like, since they are relatively short in ontogeny and life cycle from a standpoint of producing model animals for human disease.

"Mammals" in a recombinant vector that can be expressed in the mammals include the aforesaid non-human mammals and human.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean DNA that expresses abnormal GPCR of the invention and exemplified by the DNA that expresses GPCR for suppressing the function of normal GPCR of the invention.

The exogenous DNA of the present invention may be any one of those derived from a mammal of the same species as, or a different species from, the mammal as the target animal. In transferring the DNA of the present invention, it is generally advantageous to use the DNA as a DNA construct in which the DNA is ligated downstream a promoter capable of expressing the DNA in the target animal. For example, in the case of transferring the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the GPCR of the invention, there are Escherichia coli-derived plasmids, Bacillus subtilis-derived plasmids, yeast-derived plasmids, bacteriophages such as λ phage, retroviruses such as Moloney leukemia virus, etc., and animal viruses such as vaccinia virus, baculovirus, etc. Of these vectors, Escherichia coli-derived plasmids, Bacillus subtilis-derived plasmids, or yeast-derived plasmids, etc. are preferably used.

Examples of these promoters for regulating the DNA expression include (i) promoters for DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), protein chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among them, cytomegalovirus promoters, human protein elongation factor 1α (EF-1α) promoters, human and fowl β actin promoters, etc., which are capable of high expression in the whole body are preferred.

Preferably, the vectors described above have a sequence that terminates the transcription of the desired messenger RNA in the DNA transgenic animal (generally termed a terminator); for example, a sequence of each DNA derived from viruses and various mammals, and SV40 terminator of the simian virus and the like are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The translational region for normal GPCR of the invention can be obtained using as a starting material the entire genomic DNA or its portion of liver, kidney, thyroid cell or fibroblast origin from human or various mammals (e. g., rabbits, dogs, cats, guinea pigs, hamsters, rats, mice, etc.) or of various commercially available genomic DNA libraries, or using cDNA prepared by a publicly known method from RNA of liver, kidney, thyroid cell or fibroblast origin as a starting material. Also, an exogenous abnormal DNA can produce the translational region through variation of the translational region of normal GPCR of the invention obtained from the cells or tissues described above by point mutagenesis.

The translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transferred at the fertilized egg cell stage in a manner such that the DNA is certainly present in all the germinal cells and somatic cells of the target mammal. The fact that the exogenous DNA of the present invention is present in the germinal cells of the animal prepared by DNA transfer means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal in which the normal exogenous DNA of the present invention has been transferred can be passaged as the DNA-bearing animal under ordinary rearing environment, by confirming that the exogenous DNA is stably retained by crossing.

By transfer of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfer means that the DNA of the present invention is excessively present in all of the germinal cells and somatic cells thereof. The offspring of the animal that inherits the exogenous DNA of the present invention have excessively the DNA of the present invention in all of the germinal cells and somatic cells thereof.

It is possible to obtain homozygous animals having the transferred DNA in both homologous chromosomes and breed male and female of the animal so that all the progeny have this DNA in excess.

In a non-human mammal bearing the normal DNA of the present invention, the normal DNA of the present invention has expressed at a high level, and may eventually develop hyperfunction in the function of the GPCR of the invention by accelerating the function of endogenous normal DNA. Therefore, the animal can be utilized as a pathologic model animal for such a disease. For example, using the normal DNA transgenic animal of the present invention, it is possible to elucidate the mechanism of hyperfunction in the function of the GPCR of the invention and the pathological mechanism of the disease associated with the GPCR of the invention and to investigate how to treat these diseases.

Furthermore, since a mammal transferred with the exogenous normal DNA of the present invention exhibits an increasing symptom of the GPCR of the invention liberated, the animal is usable for screening of a drug for the treatment of diseases associated with the GPCR of the invention.

On the other hand, a non-human mammal having the exogenous abnormal DNA of the present invention can be passaged under normal breeding conditions as the DNA-bearing animal by confirming stable retention of the exogenous DNA via crossing. Furthermore, the exogenous DNA of interest can be utilized as a starting material by inserting the DNA into the plasmid described above. The DNA construct with a promoter can be prepared by conventional DNA engineering techniques. The transfer of the abnormal DNA of the present invention at the fertilized egg cell stage is preserved to be present in all of the germinal and somatic cells of the target mammal. The fact that the abnormal DNA of the present invention is present in the germinal cells of the animal after DNA transfer means that all of the offspring of the prepared animal have the abnormal DNA of the present invention in all of the germinal and somatic cells. Such an offspring that passaged the exogenous DNA of the present invention will have the abnormal DNA of the present invention in all of the germinal and somatic cells. A homozygous animal having the introduced DNA on both of homologous chromosomes can be acquired, and by crossing these male and female animals, all the offspring can be bred to retain the DNA.

In a non-human mammal bearing the abnormal DNA of the present invention, the abnormal DNA of the present invention has expressed to a high level, and may eventually develop the function inactive type inadaptability to the GPCR of the invention by inhibiting the functions of endogenous normal DNA. Therefore, the animal can be utilized as a pathologic model animal for such a disease. For example, using the abnormal DNA transgenic animal of the present invention, it is possible to elucidate the mechanism of the function inactive type inadaptability to the GPCR of the invention and the pathological mechanism of the disease and to investigate how to treat the disease.

More specifically, the transgenic animal of the present invention expressing the abnormal DNA of the present invention at a high level is expected to serve as an experimental model to elucidate the mechanism of the functional inhibition (dominant negative effect) of a normal the GPCR of the invention by the abnormal the GPCR of the invention in the function inactive type inadaptability of the GPCR of the invention.

A mammal bearing the abnormal exogenous DNA of the present invention is also expected to serve for screening a candidate drug for the treatment of the function inactive type inadaptability of the GPCR of the invention, since a free form of the GPCR of the invention is increased in such an animal.

Other potential applications of two kinds of the DNA transgenic animals of the present invention described above further include, for example:

(1) Use as a cell source for tissue culture;
(2) Elucidation of the relation to the GPCR of the invention that is specifically expressed or activated by the GPCR of the invention, by direct analysis of DNA or RNA in tissues of the DNA transgenic animal of the present invention or by analysis of the GPCR of the invention tissues expressed by the DNA;
(3) Research on the function of cells derived from tissues that are usually cultured only with difficulty, using cells in tissues bearing the DNA cultured by a standard tissue culture technique;
(4) Screening a drug that enhances the functions of cells using the cells described in (3) above; and,
(5) Isolation and purification of the variant the GPCR of the invention and preparation of an antibody thereto.

Furthermore, clinical conditions of a disease associated wit the GPCR of the invention, including the function inactive type inadaptability to the GPCR of the invention can be determined by using the DNA transgenic animal of the present invention. Also, pathological findings on each organ in a disease model associated with the GPCR of the invention can be obtained in more detail, leading to the development of a new method for treatment as well as the research and therapy of any secondary diseases associated with the disease.

It is also possible to obtain a free DNA-transferred cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve to identify cells capable of producing the GPCR of the invention, and to study in association with apoptosis, differentiation or propagation or on the mechanism of signal transduction in these properties to inspect any abnormality therein. Thus, the DNA transgenic animal can provide an effective research material for the GPCR of the invention and for investigation of the function and effect thereof.

To develop a drug for the treatment of diseases associated with the GPCR of the invention, including the function inactive type inadaptability to the GPCR of the invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the GPCR of the invention, using the DNA transgenic animal of the present invention or a vector capable of expressing the exogenous DNA of the present invention.

(11) Knockout animal

The present invention provides a non-human mammalian embryonic stem cell bearing the DNA of the present invention inactivated and a non-human mammal deficient in expressing the DNA of the present invention.

Thus, the present invention provides:

[1] A non-human mammalian embryonic stem cell in which the DNA of the present invention is inactivated;
[2] The embryonic stem cell according to [1], wherein the DNA is inactivated by introducing a reporter gene (e.g., β-galactosidase gene derived from *Escherichia coli*);
[3] The embryonic stem cell according to [1], which is resistant to neomycin;
[4] The embryonic stem cell according to [1], wherein the non-human mammal is a rodent;
[5] The embryonic stem cell according to [4], wherein the rodent is mouse;
[6] A non-human mammal deficient in expressing the DNA of the present invention, wherein the DNA is inactivated;
[7] The non-human mammal according to [6], wherein the DNA is inactivated by inserting a reporter gene (e.g., β-galactosidase derived from *Escherichia coli*) therein and the reporter gene is capable of being expressed under control of a promoter for the DNA of the present invention;

[8] The non-human mammal according to [6], which is a rodent;

[9] The non-human mammal according to [8], wherein the rodent is mouse; and,

[10] A method of screening a compound that promotes or inhibits (preferably inhibits) the promoter activity to the DNA of the present invention, which comprises administering a test compound to the mammal of [7] and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the GPCR of the invention (hereinafter sometimes referred to as the knockout DNA of the present invention) by substantially inactivating the activities of the GPCR of the invention encoded by the DNA (hereinafter merely referred to as ES cell).

As the non-human mammal, the same examples as described above apply.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon region thereby to disable the functions of exon, or integrating to a chromosome of the target animal by, e.g., homologous recombination, a DNA sequence that terminates gene transcription (e.g., polyA additional signal, etc.) in the intron between exons, thus inhibiting the synthesis of complete messenger RNA and eventually destroying the gene (hereinafter briefly referred to as a targeting vector). The thus-obtained ES cells to the southern hybridization analysis with a DNA sequence on or near the DNA of the present invention as a probe, or to PCR analysis with a DNA sequence on the targeting vector and another DNA sequence near the DNA of the present invention which is not included in the targeting vector as primers, to select the knockout ES cell of the present invention.

The parent ES cells to inactivate the DNA of the present invention by homologous recombination, etc. may be of a strain already established as described above, or may originally be established in accordance with a modification of the known method by Evans and Kaufman described above. For example, in the case of mouse ES cells, currently it is common practice to use ES cells of the 129 strain. However, since their immunological background is obscure, the C57BL/6 mouse or the $BDF_1$ mouse ($F_1$ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The $BDF_1$ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about $10^6$ cells; therefore, the first selection of ES cells at the early stage of culture can be based on sex identification, and male cells can be selected early, which saves a significant amount of time at the early stage of culture.

Also, second selection can be achieved by, for example, confirmation of the number of chromosomes by the G-banding method. It is usually desirable that the chromosome number of the obtained ES cells be 100% of the normal number. However, when it is difficult to obtain the cells having the normal number of chromosomes due to physical operations, etc. in the cell establishment, it is desirable that the ES cell is again cloned to a normal cell (e.g., in a mouse cell having the number of chromosomes being 2n = 40) after knockout of the gene of the ES cells.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably 5% carbon dioxide and 95% air, or 5% oxygen, 5% carbon dioxide and 90% air) in the presence of LIF (1 to 10000 U/ml) on appropriate feeder cells such as STO fibroblasts, treated with a trypsin/EDTA solution (normally 0.001 to 0.5% trypsin/0.1 to about 5 mM EDTA, preferably about 0.1% trypsin/1 mM EDTA) at the time of passage to obtain separate single cells, which are then plated on freshly prepared feeder cells. This passage is normally conducted every 1 to 3 days; it is desirable that cells be observed at the passage and cells found to be morphologically abnormal in culture, if any, be abandoned.

Where ES cells are allowed to reach a high density in mono-layers or to form cell aggregates in suspension under appropriate conditions, it is possible to differentiate the ES cells to various cell types, for example, pariental and visceral muscles, cardiac muscle or the like [M. J. Evans and M. H. Kaufman, Nature, 292, 154,1981; G. R. Martin, Proc. Natl. Acad. Sci. U.S.A., 78, 7634, 1981; T. C. Doetschman et al., Journal of Embryology Experimental Morphology, 87, 27, 1985]. The cells deficient in expression of the DNA of the present invention, which are obtained from the differentiated ES cells of the present invention, are useful for studying the GPCR of the invention in vitro or the GPCR of the invention cytologically.

The non-human mammal deficient in expression of the DNA of the present invention can be identified from a normal animal by measuring the mRNA level in the subject animal by a publicly known method, and indirectly comparing the degrees of expression.

As the non-human mammal, the same examples supra apply.

With respect to the non-human mammal deficient in expression of the DNA of the present invention, the DNA of the present invention can be made knockout by transferring a targeting vector, prepared as described above, to mouse embryonic stem cells or mouse oocytes, and conducting homologous recombination in which a targeting vector DNA sequence, wherein the DNA of the present invention is inactivated by the transfer, is replaced with the DNA of the present invention on a chromosome of a mouse embryonic stem cell or mouse embryo.

The knockout cells with the disrupted DNA of the present invention can be identified by the southern hybridization analysis using as a probe a DNA fragment on or near the DNA of the present invention, or by the PCR analysis using as primers a DNA sequence on the targeting vector and another DNA sequence at the proximal region of other than the DNA of the present invention derived from mouse used in the targeting vector. When non-human mammal stem cells are used, a cell line wherein the DNA of the present invention is inactivated by homologous recombination is cloned; the resulting clones are injected to, e.g., a non-human mammalian embryo or blastocyst, at an appropriate stage such as the 8-cell stage. The resulting chimeric embryos are transplanted to the uterus of the pseudopregnant non-human mammal. The resulting animal is a chimeric animal constructed with both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the GPCR of the invention. The individuals deficient in homozygous expression of the GPCR of the invention can be obtained from offspring of the intercross between those deficient in heterozygous expression of the GPCR of the invention.

When an oocyte is used, a DNA solution may be injected, e.g., into the prenucleus by microinjection thereby to obtain a transgenic non-human mammal having a targeting vector introduced in its chromosome. From such transgenic non-human mammals, those having a mutation at the locus of the DNA of the present invention can be obtained by selection based on homologous recombination.

As described above, the individuals in which the DNA of the present invention is rendered knockout permit passage rearing under ordinary rearing conditions, after the individuals obtained by their crossing have proven to have been knockout.

Furthermore, the genital system may be obtained and retained by conventional methods. That is, by crossing male and female animals each having the inactivated DNA, homozygous animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammalian embryonic stem cell, in which the DNA of the present invention is inactivated, is very useful for preparing a non-human mammal deficient in expression of the DNA of the present invention.

Since the non-human mammal, in which the DNA of the present invention is inactivated, lacks various biological activities derived from the GPCR of the invention, such an animal can be a disease model suspected of inactivated biological activities of the GPCR of the invention and thus, offers an effective study to investigate the causes for and therapy for these diseases.

(11a) Method of screening a compound having therapeutic/preventive effects on diseases caused by deficiency, damages, etc. of the DNA of the present invention

The non-human mammal deficient in expression of the DNA of the present invention can be employed for screening of a compound having therapeutic/preventive effects on diseases caused by deficiency, damages, etc. of the DNA of the present invention.

That is, the present invention provides a method of screening a compound having a therapeutic/preventive effect on diseases caused by deficiency, damages, etc. of the DNA of the present invention, which comprises administering a test compound to a non-human mammal deficient in expression of the DNA of the present invention and, observing and measuring a change occurred in the animal.

As the non-human mammal deficient in expression of the DNA of the present invention, which can be employed for the screening method, the same examples as given hereinabove apply.

Examples of the test compound include peptides, proteins, non-peptide . compounds, synthetic compounds, fermentation products, cell extracts, plant extracts, animal tissue extracts, blood plasma, etc. These compounds may be novel compounds or publicly known compounds.

Specifically, the non-human mammal deficient in expression of the DNA of the present invention is treated with a test compound, comparison is made with an intact animal for control and a change in each organ, tissue, disease conditions, etc. of the animal is used as an indicator to assess the therapeutic/preventive effects of the test compound.

For treating an animal to be tested with a test compound, for example, oral administration, intravenous injection, etc. are applied, and the treatment can be appropriately selected depending on conditions of the test animal, properties of the test compound, etc. Furthermore, a dose of the test compound to be administered can be appropriately selected depending on the administration methods, nature of the test compound, etc.

When a test compound is administered to a test animal in the screening method and blood sugar level of the test animal increases by at least about 10%, preferably at least 30%, more preferably at least about 50%, the test compound can be selected to be a compound having a therapeutic/preventive effect on the diseases described above.

The compound obtained using the above screening method is a compound selected from the test compounds described above and can be used as a safe and low toxic drug such as a preventive/therapeutic drug for diseases caused by deficiencies, damages, etc. of the GPCR of the invention (a preventive/therapeutic drug for diseases, e.g., anxiety, insomnia, excitement, muscular atrophy, anesthesia resistance, convulsion, dependence, schizophrenia, epilepsy, psychosomatic disorders, incontinence, neurogenic hypertension, threatened abortion, threatened premature delivery, male infertility, autonomic imbalance, etc.), a central or peripheral neural function-regulating agent, etc. Furthermore, the compound obtained using the above screening method can be used as a preventive/therapeutic drug for diseases such as cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia, palsy, etc.

In addition, compounds derived from the compound obtained by the screening described above can be used as well.

The compound obtained by the screening method may form salts and may be used in the form of salts with physiologically acceptable acids (e.g., inorganic acids, organic acids, etc.) or bases (e.g., alkali metal salts), preferably in the form of physiologically acceptable acid addition salts. Examples of such salts are salts with inorganic acids (e. g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The pharmaceutical comprising the compound obtained by the above screening method or salts thereof can be manufactured in a manner similar to the method for preparing the pharmaceutical comprising the GPCR of the invention described above.

Since the pharmaceutical preparation thus obtained is safe and low toxic, it can be administered to human or non-human mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered to the patient (as 60 kg body weight) with schizophrenia, the compound is generally administered to the patient with schizophrenia in a daily dose of about 0.1 to about 100 mg, preferably about 1.0 to about 50 mg and, more preferably about 1.0 to about 20 mg. In parenteral administration, a single dose of said compound or its salt may vary depending upon target subject, target disease, etc. When the compound or its salt is administered to the patient (as 60 kg body weight) with schizophrenia in the form of an injectable preparation, it is advantageous to administer the compound intravenously to the patient in a daily dose of about 0.01 to about 30 mg, preferably about 0.1 to about 20 mg, and more preferably about 0.1 to about 10 mg. For other animal species, the corresponding dose as converted per 60 kg body weight can be administered.

(11b) Method of screening a compound that promotes or inhibits the activity of a promoter to the DNA of the present invention

The present invention provides a method of screening a compound or its salts that promote or inhibit the activity of a promoter to the DNA of the present invention, which comprises administering a test compound to a non-human mammal deficient in expression of the DNA of the present invention and detecting the expression of the reporter gene.

In the screening method described above, an animal in which the DNA of the present invention is inactivated by introducing a reporter gene and the reporter gene is expressed under control of a promoter to the DNA of the present invention is used as the non-human mammal deficient in expression of the DNA of the present invention, which is selected from the aforesaid non-human mammals deficient in expression of the DNA of the present invention.

The same examples of the test compound apply to specific compounds used for the screening.

As the reporter gene, the same specific examples apply to this screening method. Preferably, there are used β-galactosidase (lacZ), soluble alkaline phosphatase gene, luciferase gene and the like.

Since the reporter gene is present under control of a promoter to the DNA of the present invention in the non-human mammal deficient in expression of the DNA of the present invention wherein the DNA of the present invention is substituted with the reporter gene, the activity of the promoter can be detected by tracing the expression of a substance encoded by the reporter gene.

When a part of the DNA region encoding the GPCR of the invention is substituted with, e.g., β-galactosidase gene (lacZ) derived from *Escherichia coli,* β-galactosidase is expressed in a tissue where the GPCR of the invention should originally be expressed, instead of the GPCR of the invention. Thus, the expression state of the GPCR of the invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the GPCR of the invention, or its tissue section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening method described above are compounds that are selected from the test compounds described above and that promote or inhibit the promoter activity to the DNA of the present invention.

The compound obtained by the screening method may form salts and may be used in the form of salts with physiologically acceptable acids (e.g., inorganic acids, etc.) or bases (e.g., organic acids, etc), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that promotes the promoter activity to the DNA of the present invention is useful as a central or peripheral neural function-regulating agent.

Since the compound or its salt that promotes the promoter activity to the DNA of the present invention can promote the expression of the GPCR of the invention and can promote the function of the GPCR, the compound or its salt is useful as a drug such as a preventive/therapeutic agent for diseases associated with dysfunction of the GPCR of the invention.

Since the compound or its salt that inhibits the promoter activity to the DNA of the present invention can inhibit the expression of the GPCR of the invention and can inhibit the function of the GPCR, the compound or its salt is useful as a drug such as a preventive/therapeutic agent for diseases associated with overexpression of the GPCR of the invention.

The diseases associated with dysfunction of the GPCR of the invention include, for example, anxiety, insomnia, excitement, muscular atrophy, anesthesia resistance, convulsion, dependence, schizophrenia, epilepsy, psychosomatic disorders, incontinence, neurogenic hypertension, threatened abortion, threatened premature delivery, male infertility, autonomic imbalance, cerebrovascular disorders, cerebral palsy (polio), spastic spinal paralysis, spinal vascular disorders, cervical spondylosis, ossification of posterior longitudinal ligament, multiple sclerosis, amyotrophic lateral sclerosis, spinocerebellar degeneration, traumatic sequelae (spinal cord injury, head injury), postoperative sequelae (other cerebral disorders including brain/spinal cord tumor, other myelopathies), pain, hyperesthesia, palsy, etc.

The diseases caused by overexpression of the GPCR of the invention include, for example, ataxia, paralysis, depression, delayed awakening, deficits in memory and learning, hypotension, dysuria, hypertrophy of bladder, dyspnea, dependence or sensory paralysis, etc.

In addition, compounds derived from the compound obtained by the screening described above can be used as well.

The pharmaceutical comprising the compound obtained by the above screening method or salts thereof can be manufactured in a manner similar to the method for preparing the pharmaceutical comprising the compound of changing the binding properties of the GPCR of the invention or its salt to the ligand described above.

Since the pharmaceutical preparation thus obtained is safe and low toxic, it can be administered to, for example, human or non-human mammal (e.g., rat, mouse, guinea pig, rabbit, sheep, swine, bovine, horse, cat, dog, monkey, etc.).

The dose of the compound or its salt may vary depending upon target disease, subject to be administered, route of administration, etc. For example, when the compound is orally administered to the patient (as 60 kg body weight) with schizophrenia, the compound is generally administered in a daily dose of about 0.1 to about 100 mg, preferably about 1.0 to about 50 mg and, more preferably about 1.0 to about 20 mg. In parenteral administration, a single dose of said compound or its salt may vary depending upon target subject, target disease, etc. When the compound or its salt is administered to the patient (as 60 kg body weight) with schizophrenia in the form of an injectable preparation, it is advantageous to administer the compound intravenously to the patient in a daily dose of about 0.01 to about 30 mg, preferably about 0.1 to about 20 mg, and more preferably about 0.1 to about 10 mg. For other animal species, the corresponding dose as converted per 60 kg body weight can be administered.

As stated above, the non-human mammal deficient in expression of the DNA of the present invention is extremely useful for screening the compound or its salt that promotes or inhibits the promoter activities to the DNA of the present invention and can greatly contribute to elucidation of causes for various diseases suspected of deficiency in expression of the DNA of the present invention and for the development of preventive/therapeutic drug for these diseases.

Also, a so-called transgenic animal (gene transferred animal) can be prepared by using a DNA containing the promoter region of the GPCR of the invention, ligating genes encoding various proteins at the downstream and injecting the same into oocyte of an animal. It is thus possible to synthesize the GPCR of the invention therein specifically and study its activity in vivo. When an appropriate reporter gene is ligated to the promoter site described above and a cell line that expresses the gene is established, the resulting system can be utilized as the search system for a low molecular compound having the action of specifically promoting or inhibiting the in vivo productivity of the GPCR of the invention itself.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.

DNA : deoxyribonucleic acid
cDNA : complementary deoxyribonucleic acid
A : adenine
T : thymine
G : guanine
C : cytosine
RNA : ribonucleic acid
mRNA : messenger ribonucleic acid
dATP : deoxyadenosine triphosphate
dTTP : deoxythymidine triphosphate
dGTP : deoxyguanosine triphosphate
dCTP : deoxycytidine triphosphate
ATP : adenosine triphosphate
EDTA : ethylenediaminetetraacetic acid
SDS : sodium dodecyl sulfate
Gly : glycine
Ala : alanine
Val : valine
Leu : leucine
Ile : isoleucine
Ser : serine
Thr : threonine
Cys : cysteine

Met        : methionine
Glu        : glutamic acid
Asp        : aspartic acid
Lys        : lysine
Arg        : arginine
His        : histidine
Phe        : phenylalanine
Tyr        : tyrosine
Trp        : tryptophan
Pro        : proline
Asn        : asparagine
Gln        : glutamine
pGlu       : pyroglutamic acid
*          : corresponding to termination codon
Me         methyl group
Et         : ethyl group
Bu         : butyl group
Ph         : phenyl group
TC         : thiazolidine-4(R)-carboxamido group

Substituents, protecting groups and reagents generally used in this specification are presented as the codes below.

Tos        : p-toluenesulfonyl
CHO :      formyl
Bzl        : benzyl
Cl$_2$-Bzl :   2,6-dichlorobenzyl
Bom :      benzyloxymethyl
Z          : benzyloxycarbonyl
Cl-Z       : 2-chlorobenzyloxycarbonyl
Br-Z       : 2-bromobenzyl oxycarbonyl
Boc        : t-butoxycarbonyl
DNP :      dinitrophenol
Trt        : trityl
Bum :      t-butoxymethyl
Fmoc :     N-9-fluorenyl methoxycarbonyl
HOBt :     1-hydroxybenztriazole
HOOBt :    3,4-dihydro-3-hydroxy-4-oxo-1,2,3-benzotriazine
HONB       : 1-hydroxy-5-norbornene-2,3-dicarboxyimide
DCC :      N,N'-dicyclohexylcarbodiimide

The sequence identification numbers in the sequence listing of the specification indicates the following sequence, respectively.

SEQ ID NO: 1
       This shows the base sequence of cDNA encoding rat-derived rCB7T084 of the invention.
SEQ ID NO: 2
       This shows the amino acid sequence of rat-derived rCB7T084 of the invention.
SEQ ID NO: 3
       This shows the base sequence of cDNA encoding human-derived TGR7 of the invention.
SEQ ID NO: 4
       This shows the amino acid sequence of human-derived TGR7 of the invention.
SEQ ID NO: 5
       This shows the base sequence of a primer for TGR7 used in EXAMPLE 2.
SEQ ID NO: 6
       This shows the base sequence of a primer for TGR7 used in EXAMPLE 2.
SEQ ID NO: 7
       This shows the base sequence of a probe for TGR7 used in EXAMPLE 2.

SEQ ID NO: 8

    This shows the base sequence of a primer for rCB7T084 used in EXAMPLE 2.

SEQ ID NO: 9

    This shows the base sequence of a primer for rCB7T084 used in EXAMPLE 2.

SEQ ID NO: 10

    This shows the base sequence of a probe for rCB7T084 used in EXAMPLE 2.

EXAMPLES

The present invention is described in more detail below with reference to EXAMPLES, but is not deemed to limit the scope of the present invention thereto. The genetic manipulation using Escherichia coli were performed according to the methods described in the Molecular Cloning.

EXAMPLE 1

Confirmation of the reactivity of β-alanine for rCB7T084 and TGR7

The CHO-K1 cell line was cultured in HAM F-12 medium (Invitrogen) containing 10% fetal calf serum (Invitrogen), unless otherwise indicated. A day before transfection, $4.5 \times 10^5$ cells per $10 \text{ cm}^2$ were plated, followed by incubation in a $CO_2$ incubator adjusted to a concentration of 5% $CO_2$ at 37°C for at least 15 hours. Using Lipofectamine reagent (Invitrogen), transfection was carried out by modifications of the method attached to the reagent. When a 6-well plate was used for the incubation, the following procedures were performed. First, 2 tubes each having a 1.5 ml volume were prepared and 100 μl each of Opti-MEM medium (Invitrogen) was dispensed in each tube. Next, 1 μg of the expression vector was charged in one tube and in another tube, 6 μl of Lipofectamine reagent was charged, which were mixed together. The mixture was settled for 20 minutes at room temperature. To the solution, 800 μl of Opti-MEM medium was added and the resulting solution mixture for transfection was added to the CHO-K1 cells, which had been previously washed with Opti-MEM medium. Then, the cells were incubated in a $CO_2$ incubator for 6 hours. After the incubation, the cells were rinsed with PBS (Invitrogen), then scraped using 0.05% trypsin-EDTA solution (Invitrogen) and recovered by centrifugal operation. The cells obtained were counted and diluted to $5 \times 10^4$ cells per 200 μl of the medium. The dilution was dispensed onto a Black walled 96-well plate (Costar) in 200 μl each per well, followed by incubation overnight in a $CO_2$ incubator. Various test samples were added to the CHO-K1 cells in which the receptor was transiently expressed by the transfection operation described above, whereby changes in the intracellular calcium ion level were determined using FLIPR (Molecular Device). In order to determine the changes in the intracellular calcium ion level on FLIPR, the cells were pre-teated by the following procedures. First, for the purpose of adding a fluorescent dye Fluo-3AM (DOJIN) to the cells or washing the cells immediately before the FLIPR assay, an assay buffer was prepared. A solution obtained by adding 20 ml of 1M HEPES (pH 7.4) (DOJIN) to 1000 ml of HBSS (Invitrogen) was prepared (hereinafter HBSS/HEPES solution), to which 10 ml of a solution mixture obtained by dissolving 710 mg of Probenecid (Sigma) in 5 ml of 1N NaOH and further adding 5 ml of HBSS/HEPES solution thereto was added. The resulting solution was used as the assay buffer. Next, 50 μg of Fluo-3AM was dissolved in 21 μl of DMSO (DOJIN) and an equal volume of 20% Pulronic acid (Molecular Probes) was added to and mixed with the solution. The mixture was then added to 10.6 ml of the assay buffer supplemented with 105 μl of fetal calf serum to prepare a fluorescent dye solution. The medium for the transfection-treated CHO-K1 cells was removed. Immediately thereafter, the fluorescent dye solution was dispensed in 100 μl each/well and the cells were incubated in a $CO_2$ incubator for an hour so that the fluorescent dye was taken up into the cells. The cells after the incubation was washed with the assay buffer described above and set on FLIPR. A test sample added to the receptor-expressed CHO-K1 cells was prepared using the assay buffer and set on FLIPR at the same time. Following the pre-treatment above, changes in intracellular calcium level after addition of various test samples were measured on FLIPR. It was discovered that the CHO-KI cells expressing rat rCB7T084 (FIG. 2) and its human TGR7 (FIG. 1) were specifically responsive (increase in the intracellular calcium level), when β-alanine was added in $10^{-4}$ M to $10^{-5}$ M. With the CHO-K1 cells (FIG. 3) into which the expression vector alone was introduced for control, such response was not observed. That is, it was revealed that the ligand to rCB7T084 and its human TGR7 was β-alanine.

REFERENCE EXAMPLE 1

Construction of expression vectors for rCB7T084 and TGR7

The DNA fragments encoding rCB7T084 and TGR7 were cloned, respectively, in accordance with the procedures described in WO 2000/205801 and WO 2001/83748, and the DNA fragments obtained were introduced into the SalI site and the SpeI site of pAKKO-111 vector. Thus, the respective expression vectors were constructed.

EXAMPLE 2

Analysis of tissue distribution of TGR7mRNA in human by RT-PCR

By the following procedure, cDNAs were synthesized from polyA+RNA (Clontech, Inc.) derived from various human tissues and used as templates. Following the attached manual, the reaction was carried out at 42°C using 1 μg of RNA, a random primer and SuperScriptII reverse transcriptase (GIBCO BRL, Inc.). After the reaction was completed, ethanol precipitation was performed and the precipitates were dissolved in 100 μl. RT-PCR was carried out using Sequence Detection System Prism 7700 (PE Biosystems, Inc.), where 5'-GGCTTTCGAATGCACAGGAA-3' (SEQ ID NO: 5) and 5'-CGTGGAAGCCATGCTGAAG-3' (SEQ ID NO: 6) were used as primers for amplification and detection and as TaqMan probe, 5'-(Fam)-TTCTGCATCTATATCCTCAACCTGGCGG-(Tamra)-3' (SEQ ID NO: 7) was used. For the RT-PCR solution, 0.05 μl each of the 100 μM primer solutions, 0.5 μl of 5 μM TaqMan probe and 0.5 μl of the cDNA solution prepared above were added to 12.5 μl of TaqMan Universal PCR Master Mix (PE Biosystems, Inc.), and distilled water was added thereto to make the total volume of the reaction solution 25 μl. PCR was carried out by reacting at 50°C for 2 minutes and 95°C for 10 minutes, then repeating 40 times the cycle set to include 95°C for 15 seconds and 60°C for 1 minute. The amounts of mRNA of TGR7 obtained in various human tissues were calculated as the number of copies per total RNA 25 ng (FIG. 4).

EXAMPLE 3

Analysis of tissue distribution of rCB7T084mRNA in rat by RT-PCR

By the following procedure, cDNAs were synthesized from polyA+RNA derived from various rat tissues and used as templates. Following the attached manual, the reaction was carried out at 42°C using 1 μg of RNA, a random primer and SuperScriptII reverse transcriptase. After the reaction was completed, ethanol precipitation was performed and the precipitates were dissolved in 100 μl. RT-PCR was carried out using Sequence Detection System Prism 7700 (PE Biosystems, Inc.), where 5'-TGATGAACTTCCTGGCTTCTTTC-3' (SEQ ID NO: 8) and 5'-TGTCCACCTTGAAGCACTGGTA-3' (SEQ ID NO: 9) were used as primers for amplification and detection and as TaqMan probe, 5'-(Fam) CTGTGTTCAATTCTGGCATCCCGACAA-(Tamra)-3' (SEQ ID NO: 10) was used. For the RT-PCR solution, 0.05 μl each of the 100 μM primer solutions, 0.5 μl of 5 μM TaqMan probe and 0.5 μl of the cDNA solution prepared above were added to 12.5 μl of TaqMan Universal PCR Master Mix (PE Biosystems, Inc.), and distilled water was added thereto to make the total volume of the reaction solution 25 μl. PCR was carried out by reacting at 50°C for 2 minutes and 95°C for 10 minutes, then repeating 40 times the cycle set to include 95°C for 15 seconds and 60°C for 1 minute. The amounts of mRNA of rCB7T048 obtained in various rat tissues were calculated as the number of copies per total RNA 25 ng (FIG. 5).

EXAMPLE 4

Action of suppressing the intracellular cAMP level increased after forskolin addition in TGR7- and rCB7T084-expressed CHO cells by β-alanine and L-carnosine

The TGR7- and rCB7T084-expressed CHO cells were washed with assay medium (HBSS (GibcoBRL) supplemented with 0.1% bovine serum albumin and 0.2 mM IBMX). Thereafter, the cells were incubated at 37°C for 30 minutes under 5% $CO_2$ conditions. β-Alanine ($1.0 \times 10^{-3}$ M - $3.0 \times 10^{-7}$ M) and L-carnosine ($1.0 \times 10^{-3}$ M - $3.0 \times 10^{-7}$ M) diluted with the assay medium in various concentrations were added and forskokin was then added thereto in 1 μM. The cells were incubated at 37°C for 30 minutes under 5% $CO_2$ conditions. The culture supernatant was discarded and the intracellular cAMP level was determined on a plate reader (ARVO multi-label counter, Wallac, Inc.), following the protocol of cAMP Screen Kit (Applied Biosystems, Inc.).
As a result, dose-dependent and specific decrease of the intracellular cAMP level by β-alanine (FIG. 6) and L-carnosine (FIG. 7), which had been increased by forskolin addition, was detected specifically to the CHO cells introduced with TGR7-and rCB7T084 genes, as compared to CHO cells introduced with vector alone (mock). By monitoring this activity, it was considered that endogenous ligands could be purified.

EXAMPLE 5

Setting of the screening method for the agonist and antagonist to TGR7

(1) Setting of the screening method for the agonist and antagonist to TGR7 using the changes in intracellular calcium level as an indicator

In order to set up the survey system for the agonist and antagonist to TGR7, the assay system was set up by the following procedure.

Human TGR7-expressed CHO cell line (CHO-hTGR7) prepared by publicly known methods using human TGR7 expression vector prepared in REFERENCE EXAMPLE 1 was diluted so as to contain $3 \times 10^4$ cells/100 μl. The dilution was dispensed in a Black walled 96-well plate (Costar) in 100 μl each/well, followed by incubaton overnight in a $CO_2$ incubator. Changes in intracellular calcium level were measured using FLIPR (Molecular Device), which procedures were described below.

In 21 μl of DMSO (DOJIN) 50 μg of Fluo-3AM (DOJIN) was dissolved and an equal volume of 20% Pluronic acid (Molecular Probes) was further added to and mixed with the solution. The mixture was then added to 10.6 ml of the assay buffer [which was prepared by adding 20 ml of 1M HEPES (pH 7.4) (DOJIN) to HBSS (Invitrogen) and further adding thereto 10 ml of a solution mixture, which was obtained by dissolving 710 mg of Probenecid (Sigma) in 5 ml of 1N NaOH and 5 ml of the HBSS/HEPES solution described above] supplemented with 105 μl of fetal calf serum to prepare a fluorescent dye solution. The medium in the cell plate was removed. Immediately thereafter, the fluorescent dye solution was dispensed in 100 μl each/well and the cells were incubated at 37°C in a $CO_2$ incubator for an hour so that the fluorescent dye was taken up into the cells. The cells after the incubation was washed with the assay buffer described above. Test compounds added to the cells were diluted to various concentrations using the assay buffer and the dilutions were dispensed onto the plate. For assaying the antagonist, 90 μM μM β-alanine solution (30 μM in the final concentration upon reaction) was dispensed onto a separate plate and set on FLIPR at the same time. After the foregoing pre-treatment, the changes in intracellular calcium level after the addition of test compounds were measured on FRIPR to assay the agonist action, and then β-alanine was added to assay the antagonist action. When a test compound is the agonist, the intracellular calcium level increases, and when a test compound is the antagonist, the activity of suppressing the reaction of β-alanine later added is observed. The $EC_{50}$ value was calculated from the dose-response curve using changes in fluorescent intensity value after a test compound was added.

(2) Criterion for screening the agonist candidate based on the results of FLIPR assay

Test compounds used to screen the agonist candidate were previously diluted with DMSO (Wako) to become 10 mM, which dilutions were further diluted with the assay buffer described above upon the assay to provide for use. Using these test compounds, the fluorescence intensity values were determined on the CHO cell line wherein human TGR7 was expressed (CHO-hTGR7) by the same procedure as described above and the CHO cell line wherein human histamine H1 receptor prepared by publicly known methods using human histamine H1 receptor expression vector was expressed (CHO-H1), 30 seconds or 40 seconds after the reaction commenced. In addition, when the fluorescence intensity of 100 μM β-alanine was made 100%, the relative value was calculated and the test compound showing the value of at least 50 or 100% based on CHO-hTGR7 and satisfying 25% or less based on CHO-H1 was made a candidate for human TGR7-specific agonist.

(3) Criterion for screening the antagonist candidate based on the results of FLIPR assay

Test compounds used to screen the antagonist candidate were previously diluted with DMSO (Wako) to become 10 mM, which dilutions were further diluted with the assay buffer described above upon the assay to provide for use. Subsequently to determination of the agonist activity described above, the antagonist activities to the human TGR7-expressed CHO cell line (CHO-hTGR7) and human histamine H1 receptor-expressed CHO cell line (CHO-H1) were assayed using these test compounds. For evaluation of the antagonist, the agonist (30 μM β-alanine to CHO-hTGR7, and 10 nM histamine to CHO-H1) was added 200 seconds after the reaction began, and the fluorescence intensity value was measured at 230 seconds after the reaction began. The fluorescence intensity of agonist in the absence of any TGR7 was made 100%, the relative value of the fluorescence intensity in the presence of a test compound was calculated. The test compound which satisfies the value of 50% or less to CHO-hTGR7 and 80% or more to CHO-H1 was made a candidate for human TGR7-specific antagonist. The $IC_{50}$ value was calculated from the dose-response curve using changes in fluorescent intensity value 230 seconds after the reaction began.

EXAMPLE 6

Analysis of expression of rCB7T084 in rat dorsal root ganglion

In order to examine the expression level of rCB7T084 in rat ganglion, cDNA was prepared from the total RNA of the dorsal root ganglion and spinal cord in Wister rat (8 weeks old, male) and quantification was performed by TaqMan according to the procedure of EXAMPLE 3 (FIG. 8). Expression of rCB7T084 in the dorsal root ganglion was noted higher by 390 times than in the spinal cord, confirming that rCB7T084 was highly expressed specifically in the dorsal root ganglion. Since the dorsal root ganglion is the passing point of all sensory information from the peripheral nerves, rCB7T084 is considered to take part in transduction of sensory information such as tactile sense, pressure sense, warm sense, cold sense, pain sense, kinesthetic sense, deep pain, etc.

INDUSTRIAL APPLICABILITY

The GPCR of the invention, its partial peptide or salts thereof, or the DNA encoding the GPCR of the invention or its partial peptide is useful as a preventive/therapeutic drug for diseases such as schizophrenia, etc., which are associated with abnormalities in central and/or peripheral neural functions.

By using the GPCR of the invention, its partial peptide or a salt thereof and β-alanine or L-carnosine as its ligand, compounds that change the binding properties of β-alanine or L-carnosine to the GPCR of the invention, its partial peptide, or a salt thereof can be efficiently screened.

**Claims**

1. A central or peripheral neural function-regulating agent comprising a G protein-coupled receptor protein containing the same or substantially the same amino acid sequence as the amino acid sequence represented by SEQ ID NO: 2 or SEQ ID NO: 4, its partial peptide, or a salt thereof.

2. An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising a G protein-coupled receptor protein containing the same or substantially the same amino acid sequence as the amino acid sequence represented by SEQ ID NO: 2 or SEQ ID NO: 4, its partial peptide, or a salt thereof.

3. A central or peripheral neural function-regulating agent comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide

4. An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide.

5. A diagnostic agent for central or peripheral neural function abnormalities comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide.

6. A diagnostic agent for anxiety, insomnia, excitement, muscular atrophy, anesthesia sensitivity, convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia, palsy, ataxia, paralysis, depression, delayed awakening, deficits in memory and learning, hypotension, dysuria, hypertrophy of bladder, dyspnea or sensory paralysis, comprising a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide.

7. A central or peripheral neural function-regulating agent comprising an antibody to a G protein-coupled receptor protein having the same or substantially the same amino acid sequence as the amino acid sequence represented by SEQ ID NO: 2 or SEQ ID NO: 4, its partial peptide, or a salt thereof.

8. A preventive/therapeutic agent for ataxia, paralysis, depression, delayed awakening, deficits in memory and learning, hypotension, dysuria, hypertrophy of bladder, dyspnea, dependence or sensory paralysis, comprising an antibody to a G protein-coupled receptor protein having the same or substantially the same amino acid sequence as the amino acid sequence represented by SEQ ID NO: 2 or SEQ ID NO: 4, its partial peptide, or a salt thereof.

9. A central or peripheral neural function-regulating agent comprising a polynucleotide comprising a base sequence complementary to a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence.

10. A preventive/therapeutic agent for ataxia, paralysis, depression, delayed awakening, deficits in memory and learning, hypotension, dysuria, hypertrophy of bladder, dyspnea, dependence or sensory paralysis, comprising a polynucleotide comprising a base sequence complementary to a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence.

11. A method of screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine or L-carnosine, which comprises using (1) the receptor protein, its partial peptide, or a salt thereof and (2) β-alanine or L-carnosine.

12. A kit for screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine or L-carnosine, comprising (1) the receptor protein, its partial peptide, or a salt thereof and (2) β-alanine or L-carnosine.

13. A compound or its salt that changes the binding properties of β-alanine or L-carnosine to a G protein-coupled receptor protein containing the same or substantially the same amino acid sequence as the amino acid sequence represented by SEQ ID NO: 2 or SEQ ID NO: 4, or a salt thereof, which is obtainable using the screening method according to claim 11 or the screening kit according to claim 12.

14. A method of screening an agonist or antagonist to a G protein-coupled receptor protein containing the same or substantially the same amino acid sequence as the amino acid sequence represented by SEQ ID NO: 2 or SEQ ID NO: 4, or a salt thereof, which comprises using (1) the receptor protein, its partial peptide, or a salt thereof and (2) a compound or its salt that changes the binding properties of the receptor protein or a salt thereof to β-alanine or L-carnosine.

15. A kit for screening an agonist or antagonist to a G protein-coupled receptor protein containing the same or substantially the same amino acid sequence as the amino acid sequence represented by SEQ ID NO: 2 or SEQ ID NO: 4, or a salt thereof, comprising (1) the receptor protein, its partial peptide, or a salt thereof and (2) a compound or its salt that changes the binding properties of the receptor protein or a salt thereof to β-alanine or L-carnosine.

16. A pharmaceutical comprising a compound or its salt that changes the binding properties of β-alanine or L-carnosine to a G protein-coupled receptor protein containing the same or substantially the same amino acid sequence as the amino acid sequence represented by SEQ ID NO: 2 or SEQ ID NO: 4, or a salt thereof.

17. The pharmaceutical according to claim 16, which is a central or peripheral neural function-regulating agent.

18. A method of screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine, which comprises using (1) the receptor protein, its partial peptide, or a salt thereof and (2) β-alanine.

19. A kit for screening a compound or its salt that changes the binding properties of a G protein-coupled receptor protein containing the same or substantially the same amino acid sequence as the amino acid sequence represented by SEQ ID NO: 2 or SEQ ID NO: 4, or a salt thereof to β-alanine, comprising (1) the receptor protein, its partial peptide, or a salt thereof and (2) β-alanine.

20. A compound or its salt that changes the binding properties of β-alanine to a G protein-coupled receptor protein containing the same or substantially the same amino acid sequence as the amino acid sequence represented by SEQ ID NO: 2 or SEQ ID NO: 4, or a salt thereof, which is obtainable using the screening method according to claim 18 or the screening kit according to claim 19.

21. A method of screening an agonist or antagonist to a G protein-coupled receptor protein containing the same or substantially the same amino acid sequence as the amino acid sequence represented by SEQ ID NO: 2 or SEQ ID NO: 4, or a salt thereof, which comprises using (1) the receptor protein, its partial peptide, or a salt thereof and (2) a compound or its salt that changes the binding properties of the receptor protein or a salt thereof to β-alanine.

22. A kit for screening an agonist or antagonist to a G protein-coupled receptor protein containing the same or substantially the same amino acid sequence as the amino acid sequence represented by SEQ ID NO: 2 or SEQ ID NO: 4, or a salt thereof, comprising (1) the receptor protein, its partial peptide, or a salt thereof and (2) a compound or its salt that changes the binding properties of the receptor protein or a salt thereof to β-alanine.

23. A pharmaceutical comprising a compound or its salt that changes the binding properties of β-alanine to a G protein-coupled receptor protein containing the same or substantially the same amino acid sequence as the amino acid sequence represented by SEQ ID NO: 2 or SEQ ID NO: 4, or a salt thereof.

24. The pharmaceutical according to claim 16, which is a central or peripheral neural function-regulating agent.

25. An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising an agonist to a G protein-coupled receptor protein containing the same or substantially the same amino acid sequence as the amino acid sequence represented by SEQ ID NO: 2 or SEQ ID NO: 4, or a salt thereof.

26. A preventive/therapeutic agent for ataxia, paralysis, depression, delayed awakening, deficits in memory and learning, hypotension, dysuria, hypertrophy of bladder, dyspnea, dependence or sensory paralysis, comprising an antagonist to a G protein-coupled receptor protein containing the same or substantially the same amino acid sequence as the amino acid sequence represented by SEQ ID NO: 2 or SEQ ID NO: 4, or a salt thereof.

27. A method of screening a compound having an action of changing the expression level of a G protein-coupled receptor protein having the same or substantially the same amino acid sequence as the amino acid sequence represented by SEQ ID NO: 2 or SEQ ID NO: 4 to regulate the central or peripheral neural function, which comprises using a polynucleotide containing a polynucleotide encoding the G protein-coupled receptor protein or its partial peptide.

28. A kit for screening a compound having an action of changing the expression level of a G protein-coupled receptor protein having the same or substantially the same amino acid sequence as the amino acid sequence represented by SEQ ID NO: 2 or SEQ ID NO: 4 to regulate the central or peripheral neural function, comprising a polynucleotide containing a polynucleotide encoding the G protein-coupled receptor protein or its partial peptide.

29. A compound having an action of changing the expression level of a G protein-coupled receptor protein having the same or substantially the same amino acid sequence as the amino acid sequence represented by SEQ ID NO: 2 or SEQ ID NO: 4 or its partial peptide to regulate the central or peripheral neural function, which is obtainable by using the screening method according to claim 27 or the screening kit according to claim 28.

30. A central or peripheral neural function-regulating agent comprising a compound which changes the expression level of a G protein-coupled receptor protein having the same or substantially the same amino acid sequence as the amino acid sequence represented by SEQ ID NO: 2 or SEQ ID NO: 4 or its partial peptide.

31. An antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, comprising a compound or its salt that increases the expression level of a G protein-coupled receptor protein containing the same or substantially the same amino acid sequence as the amino acid sequence represented by SEQ ID NO: 2 or SEQ ID NO: 4, or its partial peptide.

32. A preventive/therapeutic agent for ataxia, paralysis, depression, delayed awakening, deficits in memory and learning, hypotension, dysuria, hypertrophy of bladder, dyspnea, dependence or sensory paralysis, comprising a compound or its salt that decreases the expression level of a G protein-coupled receptor protein containing the same or substantially the same amino acid sequence as the amino acid sequence represented by SEQ ID NO: 2 or SEQ ID NO: 4, or its partial peptide.

33. A method of screening an agonist to a G protein-coupled receptor protein containing the same or substantially the same amino acid sequence as the amino acid sequence represented by SEQ ID NO: 2 or SEQ ID NO: 4, or a salt thereof, which comprises measuring the intracellular cAMP production suppressing activity when a test compound is brought in contact with a cell containing the receptor protein.

34. An agent for fortifying the signal transduction activity of a G protein-coupled receptor protein containing the same or substantially the same amino acid sequence as the amino acid sequence represented by SEQ ID NO: 2 or SEQ ID NO: 4, comprising β-alanine or L-carnosine.

**35.** The agent according to claim 34, which is a central or peripheral neural function-regulating agent.

**36.** The agent according to claim 34, which is an antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy.

**37.** An agent for fortifying the signal transduction activity of a G protein-coupled receptor protein containing the same or substantially the same amino acid sequence as the amino acid sequence represented by SEQ ID NO: 2 or SEQ ID NO: 4, comprising β-alanine.

**38.** The agent according to claim 37, which is a central or peripheral neural function-regulating agent.

**39.** The agent according to claim 37, which is an antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy.

**40.** A method for antianxiety, hypnosis and sedation, muscle relaxation or anesthesia enhancement, or for the prevention/treatment of convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy, which comprises administering to a mammal an effective dose of (1) a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or (3) an agonist to a G protein-coupled receptor protein having the same or substantially the same amino acid sequence as the amino acid sequence represented by SEQ ID NO: 2 or SEQ ID NO: 4, or a salt thereof.

**41.** A method of preventing/treating ataxia, paralysis, depression, delayed awakening, deficits in memory and learning, hypotension, dysuria, hypertrophy of bladder, dyspnea, dependence or sensory paralysis, which comprises administering to a mammal an effective dose of (1) an antibody to a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence, or (3) an antagonist to a G protein-coupled receptor protein having the same or substantially the same amino acid sequence as the amino acid sequence represented by SEQ ID NO: 2 or SEQ ID NO: 4, or a salt thereof.

**42.** Use of (1) a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or (3) an agonist to a G protein-coupled receptor protein having the same or substantially the same amino acid sequence as the amino acid sequence represented by SEQ ID NO: 2 or SEQ ID NO: 4, to manufacture an antianxiety agent, a hypnotic sedative, a muscle relaxant, an anesthetic fortifier or a preventive/therapeutic agent for convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia or palsy.

43. Use of (1) an antibody to a G protein-coupled receptor protein containing the same or substantially the same amino acid sequence as the amino acid sequence represented by SEQ ID NO: 2 or SEQ ID NO: 4, its partial peptide, or a salt thereof, (2) a polynucleotide comprising a base sequence complementary to a polynucleotide containing a polynucleotide encoding a G protein-coupled receptor protein having the same or substantially the same amino acid sequence as the amino acid sequence represented by SEQ ID NO: 2 or SEQ ID NO: 4, or its partial peptide, or a part of the base sequence, or (3) an antagonist to a G protein-coupled receptor protein having the same or substantially the same amino acid sequence as the amino acid sequence represented by SEQ ID NO: 2 or SEQ ID NO: 4, to manufacture a preventive/therapeutic agent for ataxia, paralysis, depression, delayed awakening, deficits in memory and learning, hypotension, dysuria, hypertrophy of bladder, dyspnea, dependence or sensory paralysis.

44. A diagnostic agent for central or peripheral neural function abnormalities comprising an antibody to a G protein-coupled receptor protein having the same or substantially the same amino acid sequence as the amino acid sequence represented by SEQ ID NO: 2 or SEQ ID NO: 4, its partial peptide, or a salt thereof.

45. A diagnostic agent for anxiety, insomnia, excitement, muscular atrophy, anesthesia sensitivity, convulsion, dependence, schizophrenia, epilepsy, psychosomatic disorder, incontinence, neurogenic hypertension, threatened abortion, threatened premature delivery, male infertility, autonomic imbalance, cerebrovascular disorder, cerebral palsy (polio), spastic spinal paralysis, spinal vascular disorder, cervical spondylosis, ossification of posterior longitudinal ligament, multiple sclerosis, amyotrophic lateral sclerosis, spinocerebellar degeneration, traumatic sequelae, postoperative sequelae, pain, hyperesthesia, palsy, ataxia, paralysis, depression, delayed awakening, deficits in memory and learning, hypotension, dysuria, hypertrophy of bladder, dyspnea or sensory paralysis, comprising an antibody to a G protein-coupled receptor protein having the same or substantially the same amino acid sequence as the amino acid sequence represented by SEQ ID NO: 2 or SEQ ID NO: 4, its partial peptide, or a salt thereof.

Fig. 1

Fig. 2

Fig. 3

## Fig.4

## Fig. 5

Copies/ng mRNA

Fig. 6

## Fig. 7

Fig. 8

**EP 1 491 207 A1**

## INTERNATIONAL SEARCH REPORT

| International application No. |
|---|
| PCT/JP03/03828 |

| A. CLASSIFICATION OF SUBJECT MATTER |
|---|
| Int.Cl$^7$ A61K38/17, 39/395, 45/00, 48/00, A61P9/00, 11/00, 13/00, 15/00, 21/00, 25/00, 43/00 |

According to International Patent Classification (IPC) or to both national classification and IPC

| B. FIELDS SEARCHED |
|---|
| Minimum documentation searched (classification system followed by classification symbols) |
| Int.Cl$^7$ A61K38/00-38/58, 39/395-39/44, 45/00, 45/00 |

Documentation searched other than minimum documentation to the extent that such documents are included in the fields searched

Electronic data base consulted during the international search (name of data base and, where practicable, search terms used)
WPI(DIALOG), BIOSIS(DIALOG), SwissProt/PIR/GeneSeq

| C. DOCUMENTS CONSIDERED TO BE RELEVANT |
|---|

| Category* | Citation of document, with indication, where appropriate, of the relevant passages | Relevant to claim No. |
|---|---|---|
| X | WO 01/70814 A2 (BAYER AG.), 27 September, 2001 (27.09.01), | 1-10,25-33, 42-45 |
| A | See, especially, Claim 17 & WO 01/70814 A3 & EP 1268548 A2 | 11-24,34-39 |
| X | WO 01/83748 A1 (TAKEDA CHEMICAL INDUSTRIES, LTD.), 08 November, 2001 (08.11.01), | 1-10,25-33, 42-45 |
| A | Particularly, Claim 30 & JP 2002-345471 A2 | 11-24,34-39 |
| A | DONG, Xinzhong et al., A Diverse Family of GPCRs Expressed in Specific Subsets of Nociceptive Sensory Neurons, Cell, 07 September, 2001 (07.09.01), Vol.106, No.5, pages 619 to 632 | 1-39,42-45 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 01 July, 2003 (01.07.03) | 29 July, 2003 (29.07.03) |

| Name and mailing address of the ISA/ Japanese Patent Office | Authorized officer |
|---|---|
| Facsimile No. | Telephone No. |

Form PCT/ISA/210 (second sheet) (July 1998)

70

## INTERNATIONAL SEARCH REPORT

| | International application No. |
|---|---|
| | PCT/JP03/03828 |

C (Continuation). DOCUMENTS CONSIDERED TO BE RELEVANT

| Category* | Citation of document, with indication, where appropriate, of the relevant passages | Relevant to claim No. |
|---|---|---|
| A | EP 1122313 A1 (TAKEDA CHEMICAL INDUSTRIES, LTD.), 08 August, 2001 (08.08.01), & WO 00/20580 A1 & CA 2344976 A & AU 9960050 A1 & JP 2000-175691 A | 1-39,42-45 |
| A | WO 01/57085 A2 (INCYTE GENOMICS, INC.), 09 August, 2001 (09.08.01), & WO 01/57085 A3 & EP 1252188 A2 | 1-39,42-45 |
| A | WO 01/48188 A1 (Helix Research Institute), 05 July, 2001 (05.07.01), & AU 2001022303 A & EP 1243648 A1 | 1-39,42-45 |
| A | WO 01/36471 A2 (ARENA PHARMACEUTICALS, INC.), 25 May, 2001 (25.05.01), & WO 01/36471 A3 & EP 1242448 A2 | 1-39,42-45 |

Form PCT/ISA/210 (continuation of second sheet) (July 1998)

## INTERNATIONAL SEARCH REPORT

| International application No. |
| --- |
| PCT/JP03/03828 |

| Box I   Observations where certain claims were found unsearchable (Continuation of item 2 of first sheet) |
| --- |

This international search report has not been established in respect of certain claims under Article 17(2)(a) for the following reasons:

1. ☒ Claims Nos.: 40, 41

   because they relate to subject matter not required to be searched by this Authority, namely:
   The inventions as set forth in claims 40 and 41 pertain to methods for treatment of the human body by therapy.

2. ☐ Claims Nos.:

   because they relate to parts of the international application that do not comply with the prescribed requirements to such an extent that no meaningful international search can be carried out, specifically:

3. ☐ Claims Nos.:

   because they are dependent claims and are not drafted in accordance with the second and third sentences of Rule 6.4(a).

| Box II   Observations where unity of invention is lacking (Continuation of item 3 of first sheet) |
| --- |

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**      ☐   The additional search fees were accompanied by the applicant's protest.

☐   No protest accompanied the payment of additional search fees.

Form PCT/ISA/210 (continuation of first sheet (1)) (July 1998)

SEQUENCE LISTING

<110> Takeda Chemical Industries, Ltd.

<120>  Novel Screening Method

<130>  P04-117PCT

<150>  JP 2002-093045
<151>  2002-03-28

<150>  JP 2002-361580
<151>  2002-12-13

<160>  10

<170>  PatentIn version 3.1

<210>  1
<211>  957
<212>  DNA
<213>  Rattus sp.

<220>
<221>  CDS
<222>  (1)..(957)
<223>  rat-derived rCB7T084

<400>  1

| atg | aac | tac | act | cct | tat | agc | agc | cca | gcc | cca | ggt | ctg | acc | atc | agc | 48 |
| Met | Asn | Tyr | Thr | Pro | Tyr | Ser | Ser | Pro | Ala | Pro | Gly | Leu | Thr | Ile | Ser | |
| 1 | | | | 5 | | | | | 10 | | | | | 15 | | |
| ccc | acc | atg | gac | cct | gtg | acc | tgg | gtt | tac | ttt | tca | gtg | aca | ttc | ctg | 96 |
| Pro | Thr | Met | Asp | Pro | Val | Thr | Trp | Val | Tyr | Phe | Ser | Val | Thr | Phe | Leu | |
| | | | 20 | | | | | 25 | | | | | 30 | | | |
| gcc | atg | gcc | acc | tgt | gtg | tgt | ggg | ata | gtg | ggc | aac | tcc | atg | gtg | att | 144 |
| Ala | Met | Ala | Thr | Cys | Val | Cys | Gly | Ile | Val | Gly | Asn | Ser | Met | Val | Ile | |

```
                  35                  40                  45
         tgg cta ctg agt ttc cac agt gtg cag agg tcc ccc ttc tgc acc tac    192
         Trp Leu Leu Ser Phe His Ser Val Gln Arg Ser Pro Phe Cys Thr Tyr
             50                  55                  60
         gtg ctc aac ctg gcg gtg gcc gac ctc ctc ttc ctg ctc tgc atg gcc    240
         Val Leu Asn Leu Ala Val Ala Asp Leu Leu Phe Leu Leu Cys Met Ala
         65                  70                  75                  80
         tcc ctg ctc agt ctg gaa aca ggg ccc ctg ctc aca gcc agc acc tcc    288
         Ser Leu Leu Ser Leu Glu Thr Gly Pro Leu Leu Thr Ala Ser Thr Ser
                         85                  90                  95
         gcc aga gtc tac gag ggg atg aag aga atc aag tac ttt gcc tac aca    336
         Ala Arg Val Tyr Glu Gly Met Lys Arg Ile Lys Tyr Phe Ala Tyr Thr
                     100                 105                 110
         gca ggc ctg agc ctg ctg acg gcc atc agc acc cag cgc tgt ctc tcc    384
         Ala Gly Leu Ser Leu Leu Thr Ala Ile Ser Thr Gln Arg Cys Leu Ser
                     115                 120                 125
         gtg ctt ttc ccc atc tgg tat aag tgc cac cgg ccc cag cac ctg tcg    432
         Val Leu Phe Pro Ile Trp Tyr Lys Cys His Arg Pro Gln His Leu Ser
                 130                 135                 140
         ggg gtg gta tgt ggt gtg ctg tgg gca ctg gcc ctc ctg atg aac ttc    480
         Gly Val Val Cys Gly Val Leu Trp Ala Leu Ala Leu Leu Met Asn Phe
         145                 150                 155                 160
         ctg gct tct ttc ttc tgt gtt caa ttc tgg cat ccc gac aaa tac cag    528
         Leu Ala Ser Phe Phe Cys Val Gln Phe Trp His Pro Asp Lys Tyr Gln
                     165                 170                 175
         tgc ttc aag gtg gac atg gtt ttc aac agt ctt atc ctg ggg atc ttc    576
         Cys Phe Lys Val Asp Met Val Phe Asn Ser Leu Ile Leu Gly Ile Phe
                     180                 185                 190
         atg ccc gtc atg gtc ctg acc agc gcc atc atc ttc atc cgc atg cga    624
         Met Pro Val Met Val Leu Thr Ser Ala Ile Ile Phe Ile Arg Met Arg
                     195                 200                 205
         aag aac agc ctg ctg cag aga cgg cag cct cgg cgg ctc tac gtg gtc    672
         Lys Asn Ser Leu Leu Gln Arg Arg Gln Pro Arg Arg Leu Tyr Val Val
                 210                 215                 220
         atc ctg act tcc gtc ctt gtc ttc ctt acc tgt tct ctg ccg ttg ggc    720
         Ile Leu Thr Ser Val Leu Val Phe Leu Thr Cys Ser Leu Pro Leu Gly
         225                 230                 235                 240
```

```
atc aac tgg ttc tta ctc tac tgg gtg gaa ctg ccg cag gcc gtg agg      768
Ile Asn Trp Phe Leu Leu Tyr Trp Val Glu Leu Pro Gln Ala Val Arg
                245                 250                 255

ctc ctg tac gtc tgc tca tca cgc ttc tcc tcg tct ttg agc agc agc      816
Leu Leu Tyr Val Cys Ser Ser Arg Phe Ser Ser Ser Leu Ser Ser Ser
                260                 265                 270

gcc aac cca gtc atc tac ttc ctc gtg ggc agc cag aag agc cac cgg      864
Ala Asn Pro Val Ile Tyr Phe Leu Val Gly Ser Gln Lys Ser His Arg
                275                 280                 285

ctg cag gag tct ctg ggt gct gtg ctg ggg cgg gca ctt cag gac gag      912
Leu Gln Glu Ser Leu Gly Ala Val Leu Gly Arg Ala Leu Gln Asp Glu
            290                 295                 300

cct gaa ggc agg gag acg cca tcc aca tgt act aat gat ggg gtc          957
Pro Glu Gly Arg Glu Thr Pro Ser Thr Cys Thr Asn Asp Gly Val
305                 310                 315
```

<210> 2

<211> 319

<212> PRT

<213> Rattus sp.


<400> 2

```
Met Asn Tyr Thr Pro Tyr Ser Ser Pro Ala Pro Gly Leu Thr Ile Ser
1               5                   10                  15

Pro Thr Met Asp Pro Val Thr Trp Val Tyr Phe Ser Val Thr Phe Leu
                20                  25                  30

Ala Met Ala Thr Cys Val Cys Gly Ile Val Gly Asn Ser Met Val Ile
                35                  40                  45

Trp Leu Leu Ser Phe His Ser Val Gln Arg Ser Pro Phe Cys Thr Tyr
            50                  55                  60

Val Leu Asn Leu Ala Val Ala Asp Leu Leu Phe Leu Leu Cys Met Ala
65                  70                  75                  80

Ser Leu Leu Ser Leu Glu Thr Gly Pro Leu Leu Thr Ala Ser Thr Ser
                85                  90                  95

Ala Arg Val Tyr Glu Gly Met Lys Arg Ile Lys Tyr Phe Ala Tyr Thr
                100                 105                 110

Ala Gly Leu Ser Leu Leu Thr Ala Ile Ser Thr Gln Arg Cys Leu Ser
```

```
                  115                 120                 125
Val Leu Phe Pro Ile Trp Tyr Lys Cys His Arg Pro Gln His Leu Ser
          130                 135                 140
Gly Val Val Cys Gly Val Leu Trp Ala Leu Ala Leu Leu Met Asn Phe
145                 150                 155                 160
Leu Ala Ser Phe Phe Cys Val Gln Phe Trp His Pro Asp Lys Tyr Gln
                  165                 170                 175
Cys Phe Lys Val Asp Met Val Phe Asn Ser Leu Ile Leu Gly Ile Phe
                  180                 185                 190
Met Pro Val Met Val Leu Thr Ser Ala Ile Ile Phe Ile Arg Met Arg
          195                 200                 205
Lys Asn Ser Leu Leu Gln Arg Arg Gln Pro Arg Arg Leu Tyr Val Val
          210                 215                 220
Ile Leu Thr Ser Val Leu Val Phe Leu Thr Cys Ser Leu Pro Leu Gly
225                 230                 235                 240
Ile Asn Trp Phe Leu Leu Tyr Trp Val Glu Leu Pro Gln Ala Val Arg
                  245                 250                 255
Leu Leu Tyr Val Cys Ser Ser Arg Phe Ser Ser Ser Leu Ser Ser Ser
                  260                 265                 270
Ala Asn Pro Val Ile Tyr Phe Leu Val Gly Ser Gln Lys Ser His Arg
          275                 280                 285
Leu Gln Glu Ser Leu Gly Ala Val Leu Gly Arg Ala Leu Gln Asp Glu
          290                 295                 300
Pro Glu Gly Arg Glu Thr Pro Ser Thr Cys Thr Asn Asp Gly Val
305                 310                 315
```

<210> 3

<211> 963

<212> DNA

<213> Homo sapiens


<220>

<221> CDS

<222> (1)..(963)

<223> human-derived TGR7


<400> 3

```
atg aac cag act ttg aat agc agt ggg acc gtg gag tca gcc cta aac      48
Met Asn Gln Thr Leu Asn Ser Ser Gly Thr Val Glu Ser Ala Leu Asn
1               5                   10                  15

tat tcc aga ggg agc aca gtg cac acg gcc tac ctg gtg ctg agc tcc      96
Tyr Ser Arg Gly Ser Thr Val His Thr Ala Tyr Leu Val Leu Ser Ser
                20                  25                  30

ctg gcc atg ttc acc tgc ctg tgc ggg atg gca ggc aac agc atg gtg     144
Leu Ala Met Phe Thr Cys Leu Cys Gly Met Ala Gly Asn Ser Met Val
            35                  40                  45

atc tgg ctg ctg ggc ttt cga atg cac agg aac ccc ttc tgc atc tat     192
Ile Trp Leu Leu Gly Phe Arg Met His Arg Asn Pro Phe Cys Ile Tyr
        50                  55                  60

atc ctc aac ctg gcg gca gcc gac ctc ctc ttc ctc ttc agc atg gct     240
Ile Leu Asn Leu Ala Ala Ala Asp Leu Leu Phe Leu Phe Ser Met Ala
65                  70                  75                  80

tcc acg ctc agc ctg gaa acc cag ccc ctg gtc aat acc act gac aag     288
Ser Thr Leu Ser Leu Glu Thr Gln Pro Leu Val Asn Thr Thr Asp Lys
                85                  90                  95

gtc cac gag ctg atg aag aga ctg atg tac ttt gcc tac aca gtg ggc     336
Val His Glu Leu Met Lys Arg Leu Met Tyr Phe Ala Tyr Thr Val Gly
                100                 105                 110

ctg agc ctg ctg acg gcc atc agc acc cag cgc tgt ctc tct gtc ctc     384
Leu Ser Leu Leu Thr Ala Ile Ser Thr Gln Arg Cys Leu Ser Val Leu
            115                 120                 125

ttc cct atc tgg ttc aag tgt cac cgg ccc agg cac ctg tca gcc tgg     432
Phe Pro Ile Trp Phe Lys Cys His Arg Pro Arg His Leu Ser Ala Trp
        130                 135                 140

gtg tgt ggc ctg ctg tgg acg ctc tgt ctc ctg atg aac ggg ttg acc     480
Val Cys Gly Leu Leu Trp Thr Leu Cys Leu Leu Met Asn Gly Leu Thr
145                 150                 155                 160

tct tcc ttc tgc agc aag ttc ttg aaa ttc aat gaa gat cgg tgc ttc     528
Ser Ser Phe Cys Ser Lys Phe Leu Lys Phe Asn Glu Asp Arg Cys Phe
                165                 170                 175

agg gtg gac atg gtc cag gcc gcc ctc atc atg ggg gtc tta acc cca     576
Arg Val Asp Met Val Gln Ala Ala Leu Ile Met Gly Val Leu Thr Pro
                180                 185                 190

gtg atg act ctg tcc agc ctg acc ctc ttt gtc tgg gtg cgg agg agc     624
```

```
Val Met Thr Leu Ser Ser Leu Thr Leu Phe Val Trp Val Arg Arg Ser
        195                 200                 205

tcc cag cag tgg cgg cgg cag ccc aca cgg ctg ttc gtg gtg gtc ctg      672
Ser Gln Gln Trp Arg Arg Gln Pro Thr Arg Leu Phe Val Val Val Leu
        210                 215                 220

gcc tct gtc ctg gtg ttc ctc atc tgt tcc ctg cct ctg agc atc tac      720
Ala Ser Val Leu Val Phe Leu Ile Cys Ser Leu Pro Leu Ser Ile Tyr
225                 230                 235                 240

tgg ttt gtg ctc tac tgg ttg agc ctg ccg ccc gag atg cag gtc ctg      768
Trp Phe Val Leu Tyr Trp Leu Ser Leu Pro Pro Glu Met Gln Val Leu
                245                 250                 255

tgc ttc agc ttg tca cgc ctc tcc tcg tcc gta agc agc agc gcc aac      816
Cys Phe Ser Leu Ser Arg Leu Ser Ser Ser Val Ser Ser Ser Ala Asn
                260                 265                 270

ccc gtc atc tac ttc ctg gtg ggc agc cgg agg agc cac agg ctg ccc      864
Pro Val Ile Tyr Phe Leu Val Gly Ser Arg Arg Ser His Arg Leu Pro
                275                 280                 285

acc agg tcc ctg ggg act gtg ctc caa cag gcg ctt cgc gag gag ccc      912
Thr Arg Ser Leu Gly Thr Val Leu Gln Gln Ala Leu Arg Glu Glu Pro
        290                 295                 300

gag ctg gaa ggt ggg gag acg ccc acc gtg ggc acc aat gag atg ggg      960
Glu Leu Glu Gly Gly Glu Thr Pro Thr Val Gly Thr Asn Glu Met Gly
305                 310                 315                 320

gct                                                                   963
Ala


<210>  4
<211>  321
<212>  PRT
<213>  Homo sapiens


<400>  4
Met Asn Gln Thr Leu Asn Ser Ser Gly Thr Val Glu Ser Ala Leu Asn
1                 5                  10                 15

Tyr Ser Arg Gly Ser Thr Val His Thr Ala Tyr Leu Val Leu Ser Ser
                20                  25                 30

Leu Ala Met Phe Thr Cys Leu Cys Gly Met Ala Gly Asn Ser Met Val
```

```
                  35                    40                    45
Ile Trp Leu Leu Gly Phe Arg Met His Arg Asn Pro Phe Cys Ile Tyr
    50                    55                    60
Ile Leu Asn Leu Ala Ala Ala Asp Leu Leu Phe Leu Phe Ser Met Ala
65                    70                    75                    80
Ser Thr Leu Ser Leu Glu Thr Gln Pro Leu Val Asn Thr Thr Asp Lys
                  85                    90                    95
Val His Glu Leu Met Lys Arg Leu Met Tyr Phe Ala Tyr Thr Val Gly
                  100                   105                   110
Leu Ser Leu Leu Thr Ala Ile Ser Thr Gln Arg Cys Leu Ser Val Leu
                  115                   120                   125
Phe Pro Ile Trp Phe Lys Cys His Arg Pro Arg His Leu Ser Ala Trp
             130                   135                   140
Val Cys Gly Leu Leu Trp Thr Leu Cys Leu Leu Met Asn Gly Leu Thr
145                   150                   155                   160
Ser Ser Phe Cys Ser Lys Phe Leu Lys Phe Asn Glu Asp Arg Cys Phe
                  165                   170                   175
Arg Val Asp Met Val Gln Ala Ala Leu Ile Met Gly Val Leu Thr Pro
                  180                   185                   190
Val Met Thr Leu Ser Ser Leu Thr Leu Phe Val Trp Val Arg Arg Ser
                  195                   200                   205
Ser Gln Gln Trp Arg Arg Gln Pro Thr Arg Leu Phe Val Val Val Leu
             210                   215                   220
Ala Ser Val Leu Val Phe Leu Ile Cys Ser Leu Pro Leu Ser Ile Tyr
225                   230                   235                   240
Trp Phe Val Leu Tyr Trp Leu Ser Leu Pro Pro Glu Met Gln Val Leu
                  245                   250                   255
Cys Phe Ser Leu Ser Arg Leu Ser Ser Ser Val Ser Ser Ser Ala Asn
                  260                   265                   270
Pro Val Ile Tyr Phe Leu Val Gly Ser Arg Arg Ser His Arg Leu Pro
             275                   280                   285
Thr Arg Ser Leu Gly Thr Val Leu Gln Gln Ala Leu Arg Glu Glu Pro
             290                   295                   300
Glu Leu Glu Gly Gly Glu Thr Pro Thr Val Gly Thr Asn Glu Met Gly
305                   310                   315                   320
Ala
```

<210> 5
<211> 20
<212> DNA
<213> Artificial Sequence

<220>
<223> Primer

<400> 5
ggctttcgaa tgcacaggaa                    20

<210> 6
<211> 19
<212> DNA
<213> Artificial Sequence

<220>
<223> Primer

<400> 6
cgtggaagcc atgctgaag                     19

<210> 7
<211> 28
<212> DNA
<213> Artificial Sequence

<220>
<223> Probe, labeled 5'-terminal with FAM and 3'-terminal with TAMRA

<400> 7
ttctgcatct atatcctcaa cctggcgg           28

<210> 8
<211> 23
<212> DNA
<213> Artificial Sequence

```
<220>
<223> Primer


<400> 8
tgatgaactt cctggcttct ttc                    23


<210> 9
<211> 22
<212> DNA
<213> Artificial Sequence


<220>
<223> Primer


<400> 9
tgtccacctt gaagcactgg ta                     22


<210> 10
<211> 27
<212> DNA
<213> Artificial Sequence


<220>
<223> Probe, labeled 5'-terminal with FAM and 3'-terminal with TAMRA


<400> 10
ctgtgttcaa ttctggcatc ccgacaa                27
```